# NEW EUROPEAN PATENT SPECIFICATION

(11) **EP 1 208 231 B2**
(45) Date of publication and mention of the opposition decision: **30.12.2009**
(45) Mention of the grant of the patent: 10.01.2007
(21) Application number: 00936765.7
(22) Date of filing: 04.05.2000
(51) Int. Cl.: C12Q 1/68

(54) **MEANS FOR DETECTING AND TREATING PATHOLOGIES LINKED TO FGFR3**
MITTEL ZUM NACHWEIS UND ZUR BEHANDLUNG VON KRANKHEITEN DIE IN VERBINDUNG STEHEN MIT FGFR3
MOYENS DE DETECTION ET DE TRAITEMENT DES PATHOLOGIES ASSOCIEES AU FGFR3

(30) Priority: 05.05.1999 US 132705 P
(43) Date of publication of application: 29.05.2002
(73) Proprietor: Institut Curie, 75248 Paris Cedex 05 (FR); CENTRE NATIONAL DE LA RECHERCHE SCIENTIFIQUE (CNRS), 75794 Paris Cedex 16 (FR)
(72) Inventor: CAPPELLEN, David, F-14810 Merville-Franceville (FR); CHOPIN, Dominique, F-94000 Creteil (FR); RADVANYI, François, F-92260 Fontenay-aux-Roses (FR); RICOL, David, F-75013 Paris (FR); THIERY, Jean-Paul, F-75013 Paris (FR)
(74) Representative: Warcoin, Jacques
(86) International application number: PCT/EP2000/004591
(87) International publication number: WO 2000/068424

(56) References cited:
- CHESI, M. ET AL.: "Frequent translocation t(4;14)(p16.3;q32.3) in multiple myeloma is associated with increased exxpression and activating mutations of fibroblast growth factor receptor 3" NATURE GENETICS, vol. 16, no. 3, 1997, pages 260-264, XP001030990
- WEBSTER, M. K. ET AL.: "Enhanced Signaling and Morphological Transformation by a Membrane-Localized Derivative of the Fibroblast Growth Factor Receptor 3 Kinase Domain" MOL. CELL. BIOL., vol. 17, no. 10, 1997, pages 5739-5747, XP002181674
- LI, Z. H. ET AL.: "An activating mutation of the myeloma associated oncogene fibroblast growth factor receptor 3 (FGFR3) has hematopoietic transforming potential in mice" BLOOD, vol. 92, no. 10, Suppl 1 (Part 1 of 2), 15 November 1998 (1998-11-15), page 383A XP001030987
- PLOWRIGHT, E.E. ET AL.: "An activating mutation of the myeloma associated oncogene fibroblast growth factor receptor 3 (FGFR3) promotes interleukin-6 (IL-6) independence and upregulation of bcl-xL" BLOOD, vol. 92, no. 10, Suppl 1 (Part 1 of 2), 15 November 1998 (1998-11-15), page 383A XP002181675
- CAPPELLEN, D. ET AL.: "Frequent activating mutations of FGFR3 in human bladder and cervix carcinomas" NATURE GENETICS, vol. 23, September 1999 (1999-09), pages 18-20, XP002181676
- FENG ET AL.: CANCER RESEARCH, vol. 57, no. 23, 1 December 1997 (1997-12-01), pages 5369-5378,
- DENG ET AL: CELL, vol. 84, no. 6, 22 March 1996 (1996-03-22), pages 911-921,
- WANG ET AL.: PROCEEDINGS OF TEH NATIONAL ACADEMY OF SCIENCES USA, vol. 96, no. 8, 13 April 1999 (1999-04-13), pages 4455-4460,
- SU ET AL: NATURE, vol. 386, no. 6622, 20 March 1997 (1997-03-20), pages 288-292,
- CAPELLEN ET AL.: 'Frequent activating mutations of FGFR3 in human bladder and cervix carcinomas' NATURE GENETICS vol. 23, September 1999, pages 18 - 19
- WU ET AL.: 'Somatic mutations of fibroblast growth factor receptor 3 (FGFR3) are uncommon in carcinomas of the uterine cervix' ONCOGENE vol. 19, 2000, pages 5543 - 5546
- KAROUI ET AL.: 'No evidence of somatic FGFR3 mutation in various types of carcinoma' ONCOGENE vol. 20, 2001, pages 5059 - 5061
- BERNARD-PIERROT ET AL.: 'Oncogenic properties of the mutated forms of fibroblast growth factor receptor 3b' CARCINOGENESIS vol. 27, no. 4, 2006, pages 740 - 747
- CHELLAIAH ET AL.: 'Fibroblast Growth Factor Receptor (FGFR) 3' THE JOURNAL OF BIOLOGICAL CHEMISTRY vol. 269, 15 April 1994, pages 11620 - 11627
- FENG ET AL.: 'Fibroblast Growth Factor Receptor 2 Limits and Receptor 1 Accelerates Tumorigenicity of Prostate Epithelial Cells' CANCER RESEARCH vol. 57, 01 December 1997, pages 5369 - 5378
- JOHNSON AND WILLIAMS: 'Structural and Fuctional Diversity in the FGF Receptor Multigene Family' ADVANCES IN CANCER RESEARCH vol. 60, 1993, pages 1 - 41
- THIERY AND CHOPIN: 'Epithelial cell plasticity in development and tumor progression' CANCER AND METASTASIS REVIEWS vol. 18, 1999, pages 31 - 42
- YEE ET AL.: 'Analysis of Fibroblast Growth Factor Receptor 3 S249C Mutation in Cervical Carcinoma (Brief Communications)' JOURNAL OF THE NATIONAL CANCER INSTITUTE vol. 92, no. 22, 15 November 2000, pages 1848 - 1849

## Description

The invention relates to means, i.e. method and drugs, for detecting and treating, respectively, pathologies linked to FGFR3 and/or to the FGFR3 pathway.

Fibroblast growth factor receptor 3 (FGFR3) belongs to a family of structurally related tyrosine kinase receptors (FGFRs 1-4) encoded by four different genes. These receptors are glycoproteins composed of two to three extracellular immunoglobulin (Ig)-like domains, a transmembrane domain and a split tyrosine-kinase domain. Alternative mRNA splicing results in many different receptors variants. Isoforms FGFR3-IIIb and FGFR3-IIIc result from a mutually exclusive splicing event in which the second half of the juxtamembrane Ig-like domain is encoded either by the 151 nucleotides long exon 8 (IIIb variant) or the 145 nucleotides long exon 9 (IIIc variant).

Specific point mutations in the *FGFR3* gene which affect different domains of the protein are associated with autosomal dominant human skeletal disorders such as hypochondroplasia, achondroplasia, severe achondroplasia with developmental delay and acanthosis nigricans and thanatophoric dysplasia. Several reports have demonstrated that these mutations lead to constitutive activation of the receptor. Taking into account this result, together with the skeletal overgrowth observed in mice homozygous for null alleles of *Fgfr3*, FGFR3 appears as a negative regulator of bone growth.

In contrast with this inhibitory role, an oncogenic role has been proposed for *FGFR3* in multiple myeloma (MM) development. In this malignant proliferation of plasma cells, a t(4;14)(p16.3;q32.3) chromosomal translocation with breakpoints located 50 to 100 Kb centromeric to *FGFR3* is present in 20-25% of the cases and is associated with overexpression of FGFR3.

In very rare cases (2 out of 12 MM cell lines and 1 out of 85 primary MM tumours), activating mutations of *FGFR3* previously identified in human skeletal disorders have been found, but always accompanied by the t(4;14)(p16.3;q32.3) translocation.

By investigating various cancers, the inventors have surprisingly found a role for FGFR3 in cancers originating from epithelial tissues, carcinomas.

The involvement of FGFR3 in such solid tumour development is linked to a constitutional activation : it may be activated by an autocrinal loop (ligand self-production) and/or by activating mutations in *FGFR3.* Surprisingly, such mutations are found in primary tumours and are somatic mutations (genomic DNA mutations).

So far, the only FGFR3 isoform which has been identified in epithelium is the FGFR3-IIIb isoform.

The invention thus relates to a method for detecting human bladder carcinomas.

According to another aspect, the invention also relates to the use of inhibitory anti-FGFR3 antibodies for the manufacture of drugs capable of treating human bladder carcinomas.

Transgenic animals enabling the efficiency of such drugs to be tested are described.

The method of the invention for detecting human bladder carcinomas in a biological sample selected from bladder tissue and urine comprises identifying FGFR3 activating mutations.

Standard methods can apply for such an identification such as immunohistochemistry, or detection of the corresponding RNA, DNA, and encoded protein contained in said sample, particularly after extraction thereof. A common way for such a detection comprises amplifying by PCR, RT-PCR or RT-PCR SSCP (single strand conformation polymorphism) with *FGFR3* specific primers and revealing the amplification products according to the usual methods. A corresponding embodiment is exemplified in the examples given hereinafter. Another common way comprises the use of antibodies and the detection of the antigen-antibody reaction with appropriate labelling.

The activating function of a mutation can be determined by observation of activating signals such as receptor phosphorylation, cell proliferation (e.g. thymidine incorporation) or indirect effects such as calcium influx, phosphorylation of target sequences.

More particularly, said identification comprises screening for single nucleotide mutation(s) in the genomic DNA and/or its products, i.e. RNA, protein, the term "product" also encompassing cDNA.

Particularly, said method comprises screening for mutations creating cysteine residues in the extracellular or transmembrane domains of the receptor.

Alternatively, or in combination with the foregoing embodiment, it comprises screening for mutations resulting in at least one amino-acid substitution in the kinase domain of the receptor.

More particularly, the method of the invention comprises screening for mutation(s) in exon 7, encoding the junction between immunoglobulin-like domains II and III of FGFR3, in exon 10, encoding the transmembrane domain, in exon 15, encoding the tyrosine kinase domain I, and/or in the exon encoding the C-terminal part.

Advantageously, the method of the invention comprises screening for missense mutations such as implicated in thanatophoric dysplasia, NSC, achondroplasia, saddan, or hypochondroplasia.

Such FGFR3 activating mutations notably comprise R248C, S249C, G372C, S373C, Y375C, K652E, K652M, J809G, J809C, J809R, J809L, P250R, G377C, G382R, A393E, N542K (codons are numbered according to FGFR3-IIIb cDNA open reading frame).

The following FGFR3 activating mutations will be particularly identified : R248C, S249C, G372C, K652E and Y375C.

Said method is useful for detecting human bladder carcinomas. A major issue in superficial bladder cancer is to distinguish tumours which will progress from those which will not. Insights into the genetic and epigenetic alterations involved in bladder cancer is expected to provide useful information to facilitate this distinction. In that respect, the invention provides means to resolve the dilemma between a bladder-sparing strategy versus cystectomy and will contribute to a more individualised intravesical instillation and endoscopic monitoring policy.

Indeed, as shown by the results given in the examples, *FGFR3* appears to be a major oncongene in Ta, T 1 bladder carcinomas. The *FGFR3* activating mutations appear to be frequently associated with tumours that do not progress. Multivariate analysis showed that *FGFR3* mutation status remained a statistically significant predictor of good outcome. *FGFR3* mutations thus provide clear-cut information, which may complement stage and grade. The use of these activating mutations alone and/or in combination with other predictors of tumour aggressiveness will then provide relevant prognostic information.

Said method could also be used for detecting for example cervix, lung, breast, colon, skin cancers.

The method of detection according to the invention applies to the diagnostic of human bladder carcinomas, as well to the prognosis, or the follow-up of the efficiency of a therapy.

Said method will advantageously be performed by using kits comprising the appropriate reagents and a notice of use.

It is herein disclosed the manufacture of drugs having an anti-proliferative effect on carcinoma cells. Such drugs comprise, as active principle(s) agent(s) which act by inhibition of FGFR3 DNA synthesis or by inhibition of its expression products (RNA, proteins). Particularly, such drugs contain tyrosine kinase inhibitors specific for FGFR3.

According to another aspect, the present invention relates to inhibitory antibodies directed against FGFR3, and particularly against at least one extracellular Ig-like domain thereof. Advantageously said inhibitory antibodies are specific for FGFR3-IIIb. Preferred inhibitory antibodies are monoclonal ones, and particularly antibodies modified so that they do not induce immunogenic reactions in a human body (e.g. humanized antibodies). The inhibitory antibodies of the invention are used for the manufacture of medicaments for treating human bladder carcinomas.

It is also described other appropriate inhibitors comprising antisense oligonucleotides directed against a wild or mutated *FGFR3* isoform.

The administration and the posology of said inhibitors will be determined by the one skilled in the art depending on the carcinoma to be treated, the weight and age of the patient. For example, antibodies will be administered by the injectable route.

Here it is described means of great interest for detecting and treating carcinomas, taking into account the fact that cancers originating from epithelial tissues (carcinomas) represent approximately 90 % of malignant neoplasms.

Disclosed are cell lines capable of expressing FGFR3 mutated forms. Particularly, disclosed are FGFR3 S249C mutated forms. T24 cell lines constitutively expressing FGFR3 S249C mutated forms and HeLa cell lines expressing FGFR3 S249C mutated forms in an inducible manner have thus been obtained (for example see ref.(6)).

By injecting such cell lines to nude mice, an increased tumorigenicity was observed.

Such cell lines are useful *in vitro* (follow up of the receptor phosphorylation) or *in vivo* (examination of the tumorigenicity of nude mice) to study the inhibitory effect against FGFR3.

Cell lines transfected with FGFR2, FGFR1 or FGFR4 are particularly useful for studying the specificity of inhibitors to be tested.

It is also disclosed constructions capable of expressing by transgenesis a FGFR3 mutated form in epitheliums and the transgenic animals thus obtained which are characterized by the fact that they comprise such constructions.

Examples of constructions intended for injection in animal germinal cells comprise a keratin promoter, particularly keratin 14 promoter and cDNA of mutated FGFR3.

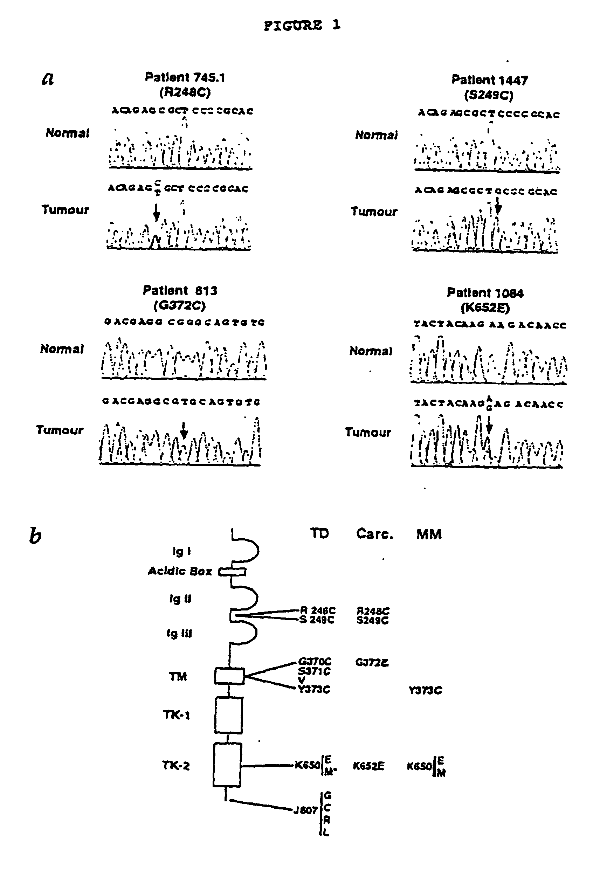

Other advantages and characteristics of the invention will be given in the following examples wherein it will be referred to
- figures 1A - 1B which give FGFR3-IIIb gene activating mutations in primary tumours,

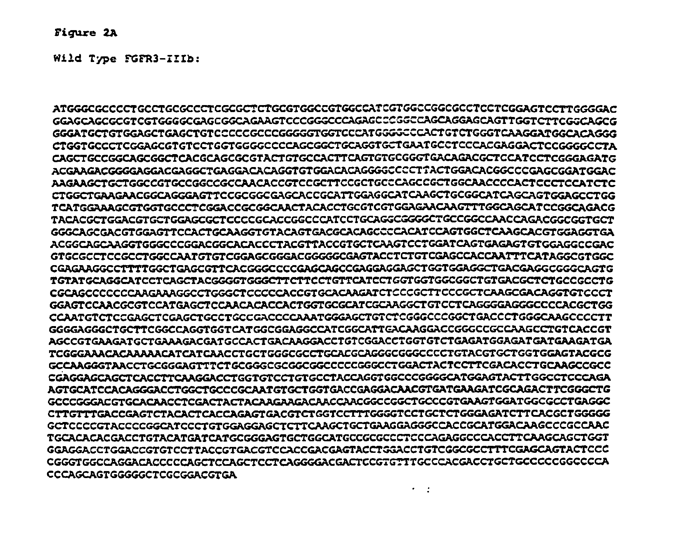

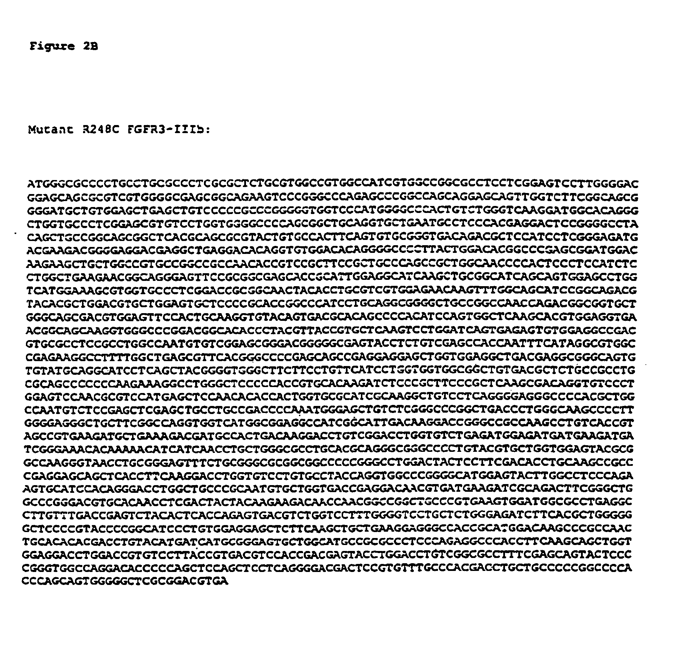

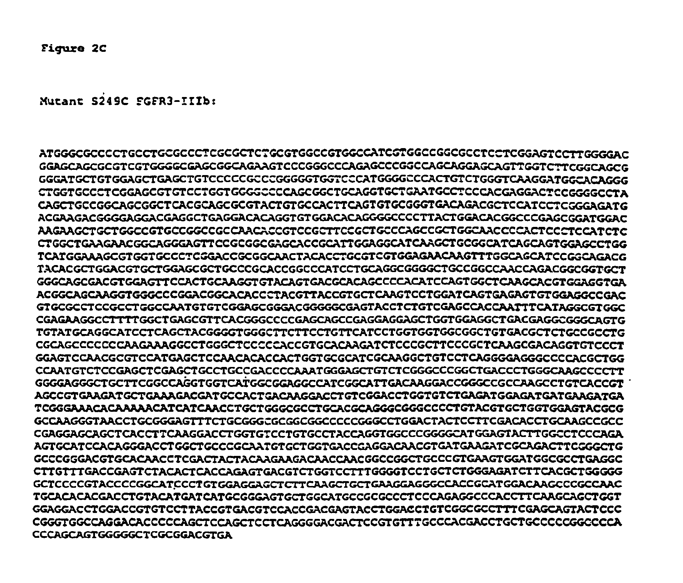

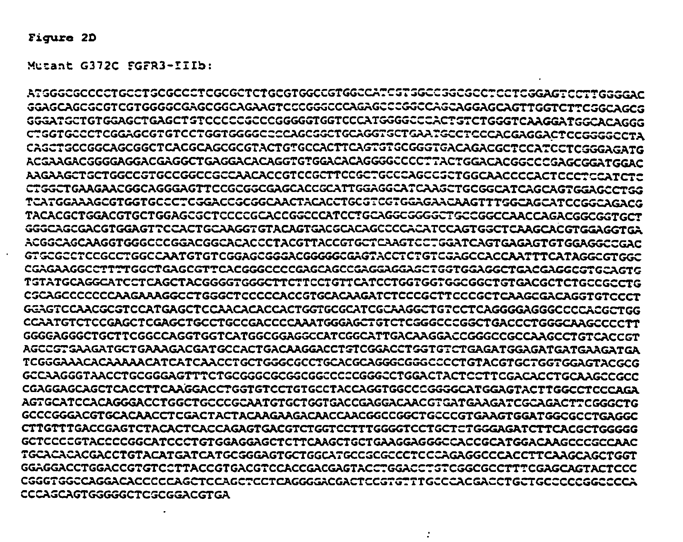

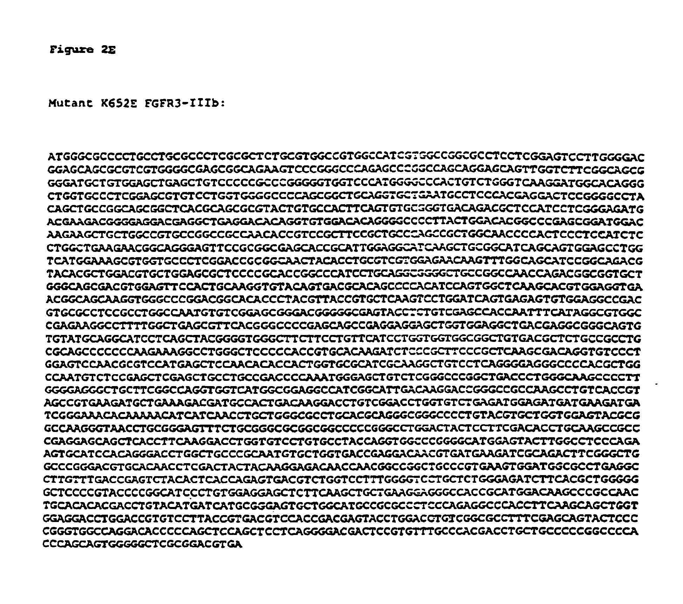

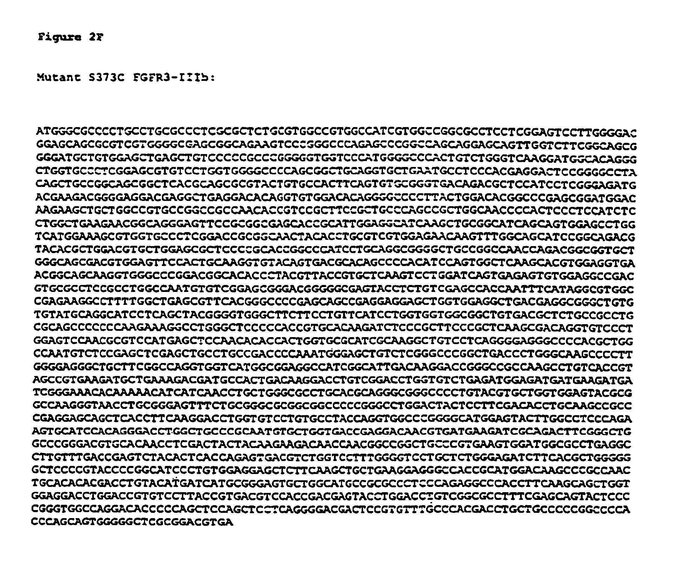

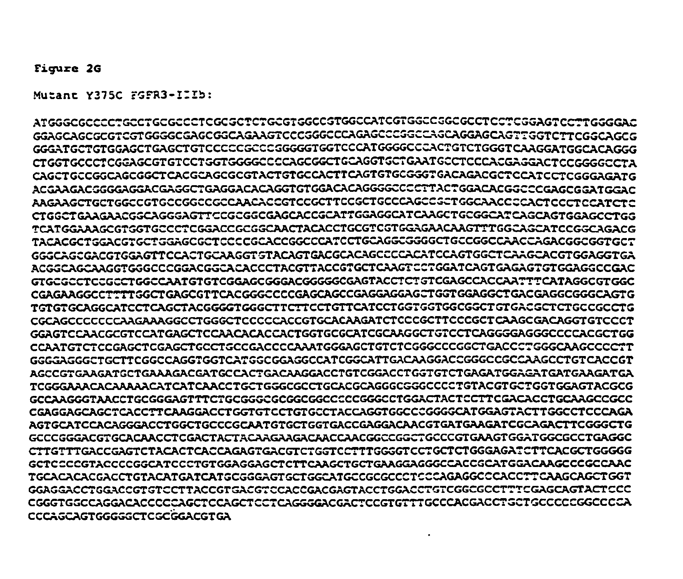

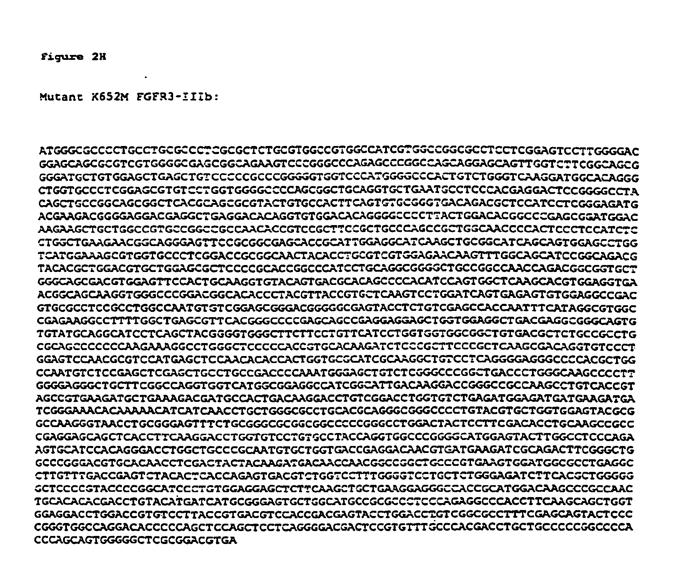

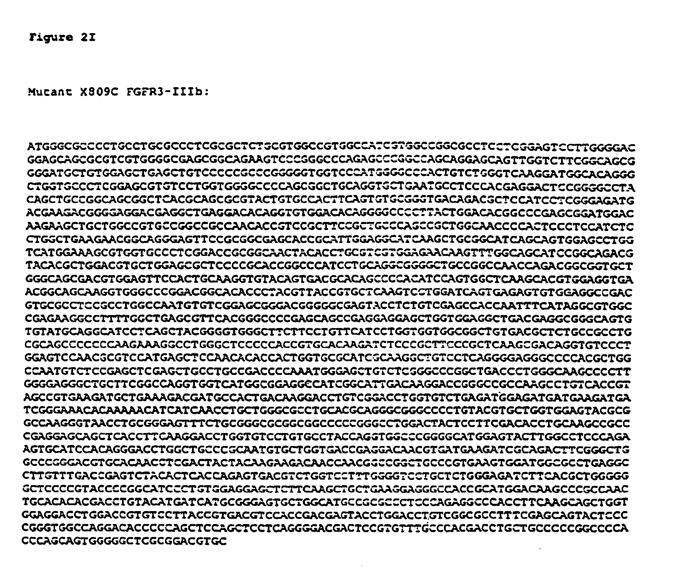

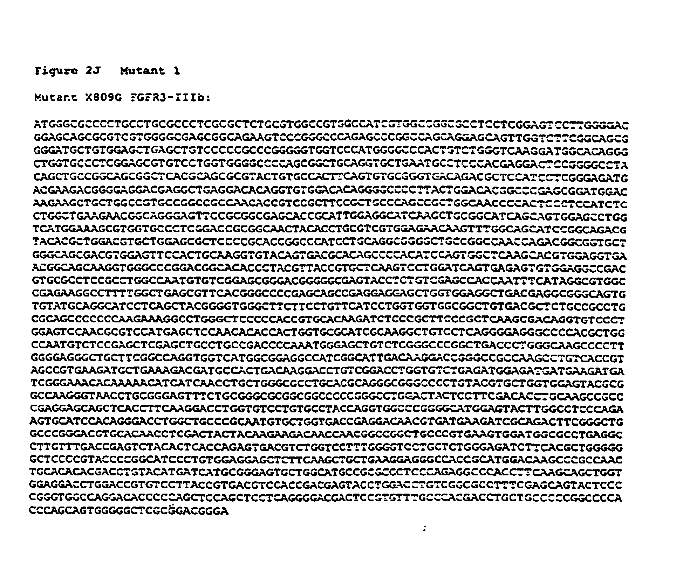

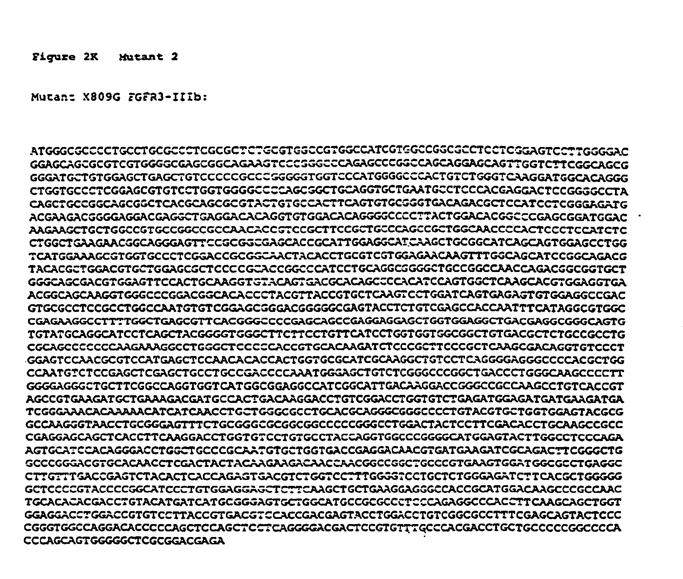

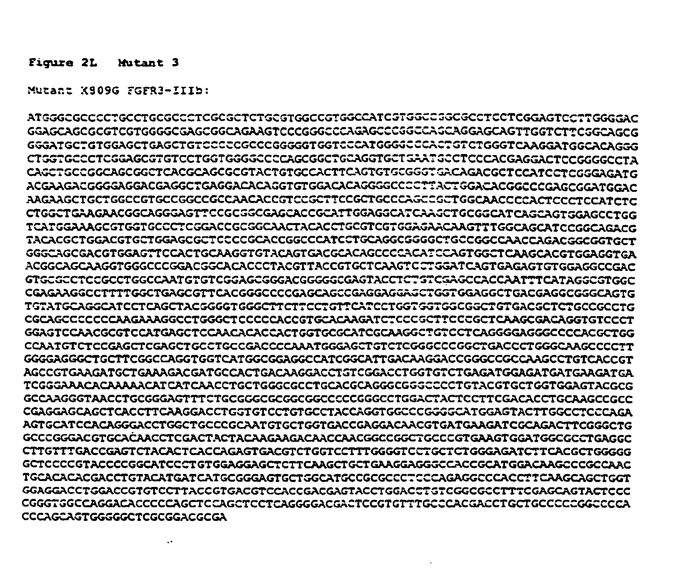

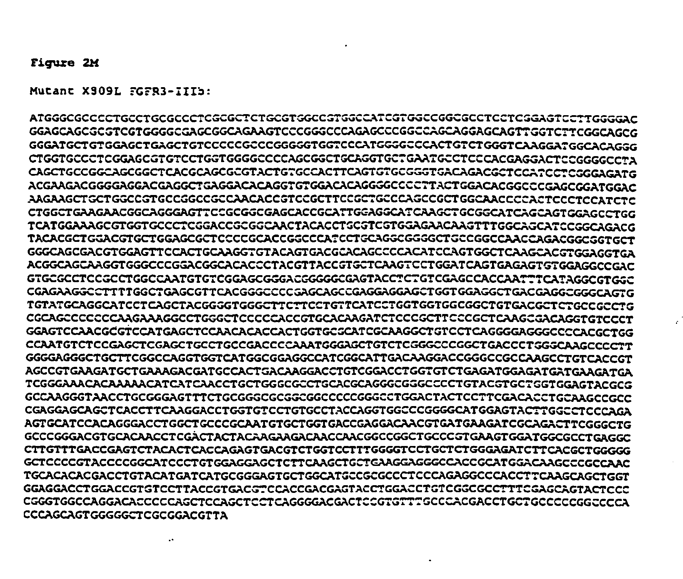

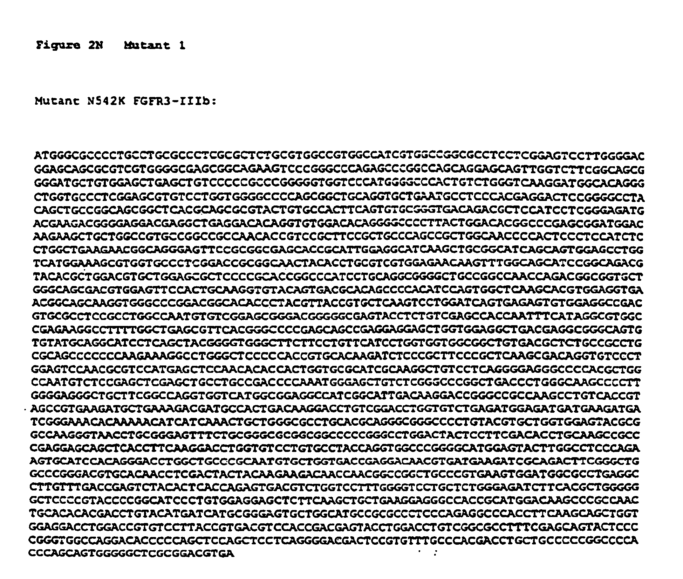

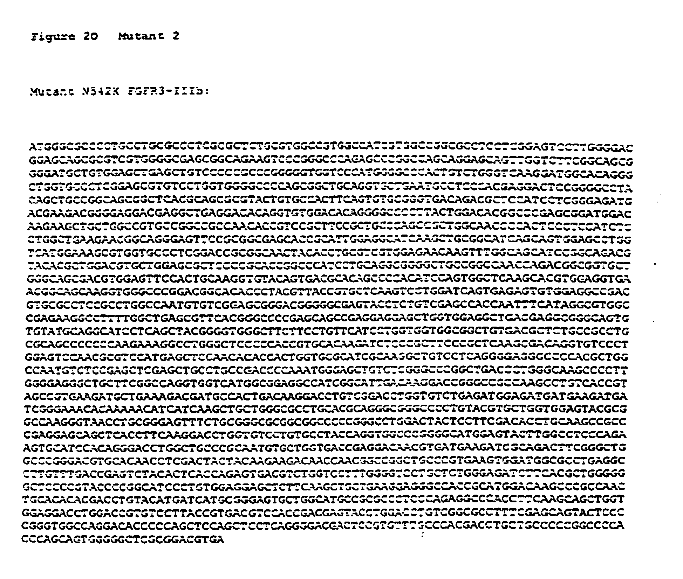

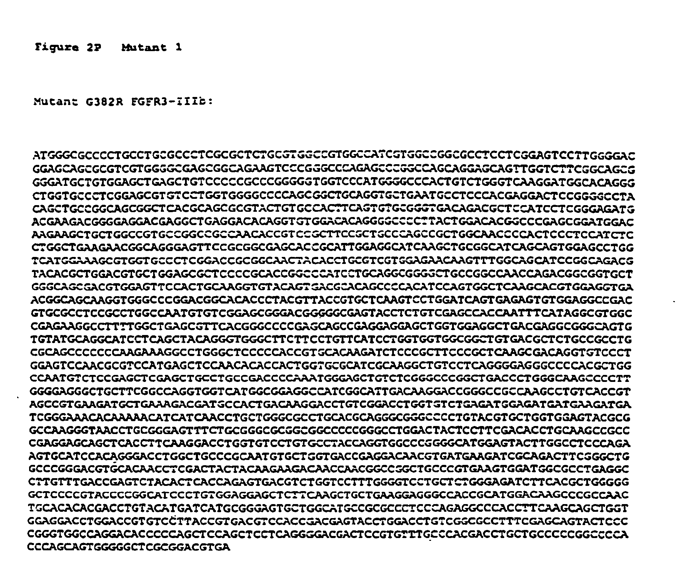

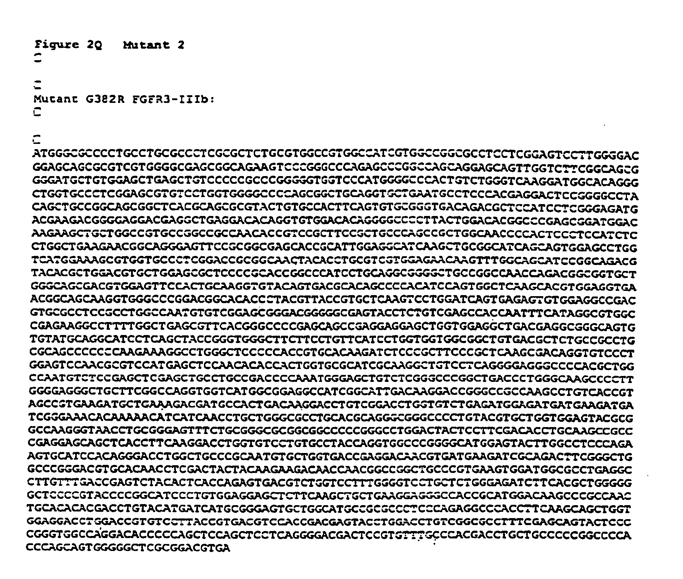

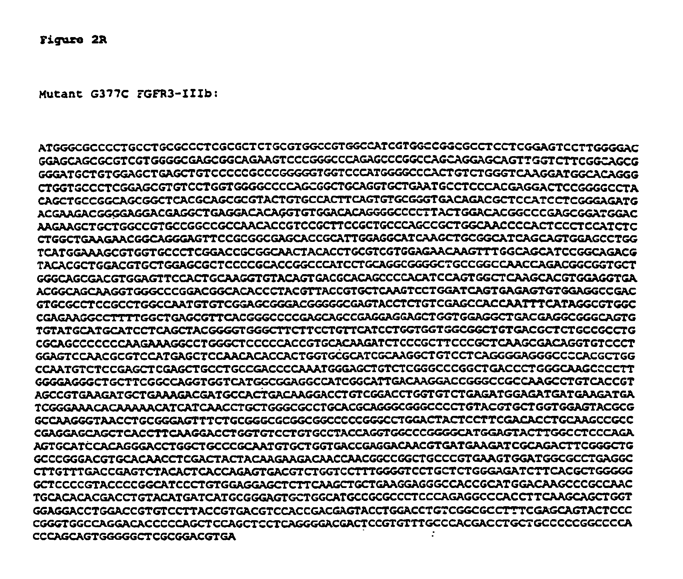

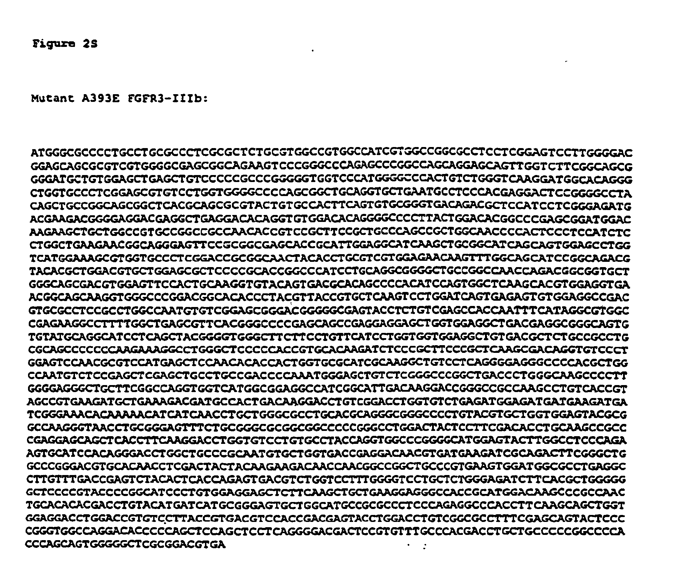

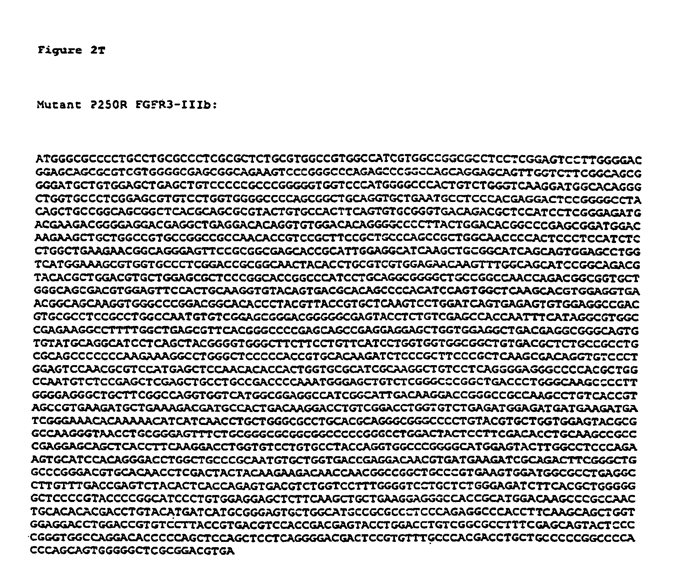

- figures 2A - 2E which refer to FGFR3-IIIb wild (2A) and mutated pro-oncogenic (2B-2T) sequences. It will be noted that the sequences of figures 2B to 2T, as such, enter into the scope of the invention. There may be silent polymorphisms all along the sequence, so there may be in fact several possible sequences for each mutant, and

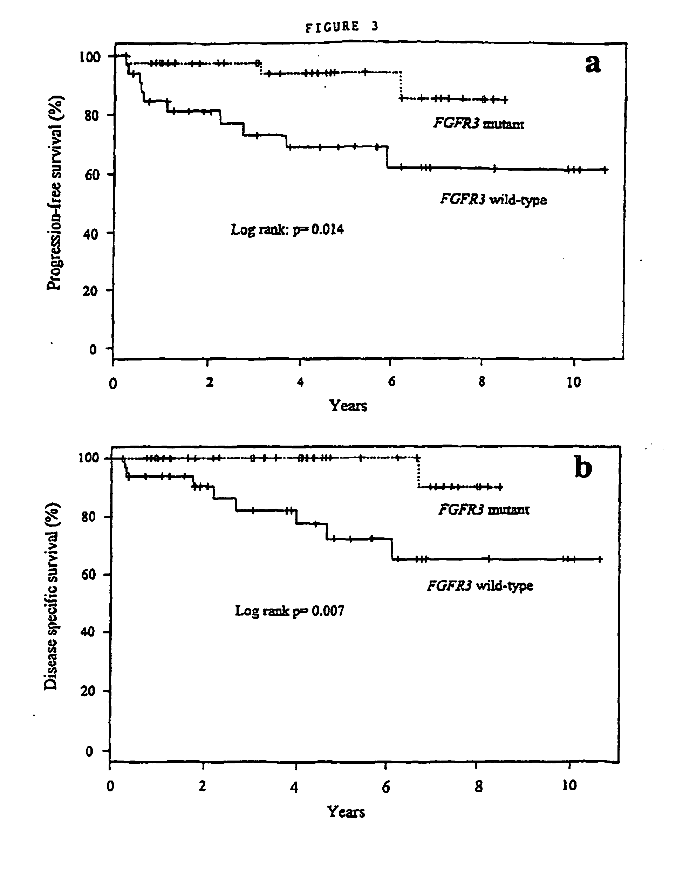

- figures 3a and 3b which respectively represent a) Kaplan-Meier progression-free survival curves according to *FGFR3* mutations (dotted line: mutated *FGFR3,* solid line: non-mutated *FGFR3;* log rank test p=0.014) ; b) Kaplan-Meier disease-specific survival curves according to *FGFR3* mutations (dotted line: mutated *FGFR3,* solid line: non-mutated *FGFR3;* log rank test p=0.007)

### Example 1 : FGFR3 gene mutations in bladder and cervix carcinomas

*FGFR3-IIIb* and *FGFR3-IIIc* transcript levels were examined by reverse transcription-polymerase chain reaction (RT-PCR) in 76 primary bladder carcinomas and 29 primary invasive cervical carcinomas.

*FGFR3-IIIb,* the sole isoform to be significantly expressed, was detected in 72 out of 76 (94%) bladder carcinomas and 27 out of 29 (93%) cervical carcinomas.

A PCR-SSCP analysis was then conducted on both reverse transcribed RNA and genomic DNA to screen for *FGFR3* coding sequence variants in 26 bladder and 12 cervix cancers expressing the gene. The results are illustrated in figures 1a and 1b which gives the identification of *FGFR3* gene mutations in human carcinomas :
- a: gives the identification of somatic mutations by direct sequencing of PCR products. Normal constitutional DNA; Tumour, tumour DNA.
- b: gives FGFR3 mutations associated with squeletal disorders and cancers.

The schematic structure of FGFR3 is depicted (Ig I-III, immunoglobulin like domains ; TM, transmembrane domain ; TK-1 and -2, tyrosine kinase domains) and the locations of the known human missense mutations associated with thanatophoric dysplasia (TD) and severe achondroplasia (SADDAN), bladder and cervix carcinomas (carc.) and multiple myeloma (MM) are indicated. Usual amino acid abbreviations are used to point out the mutation found in each pathological situation. The mutations at codon 807 incriminated in TD replaces a Stop codon (J) by an amino acid (G, C, R or L) and the mRNA thus continues to be translated until another in-frame Stop codon is reached 423 nucleotides downstream thus leading to a 141 amino acid longer protein.

Abnormally migrating bands were observed for certain samples (Fig. la) and direct sequencing of PCR products revealed single nucleotide substitutions in 9 out 26 bladder carcinomas (35 %) and 3 out of 12 (25 %) cervix carcinomas (Fig. 1b and table 1).

**Table 1**

| **Summary of FGFR3 gene mutations in primary bladder and cervix cancers** | | | | | |
|---|---|---|---|---|---|
| Sample | Histopathol. | Codon | Nt Position | Mutation | Predicted effect |
| 1447, bladder | carc., Ta G2 | 249 | 746 | TCC to TGC | Ser to Cys |
| 342, bladder | carc., Tla G1 | 249 | 746 | TCC to TGC | Ser to Cys |
| 813, bladder | carc., Tla G1 | 372 | 1114 | GGC to TGC | Gly to Cys |
| 1393.1, bladder | carc., Tla G3 | 249 | 746 | TCC to TGC | Ser to Cys |
| 506, bladder | carc., Tlb G2 | 372 | 1114 | GGC to TGC | Gly to Cys |
| 1084, bladder | carc., Tlb G3 | 652 | 1954 | AAG to GAG | Lys to Glu |
| 745.1, bladder | carc., T2 G3 | 248 | 742 | CGC to TGC | Arg to Cys |
| 1077, bladder | carc., T3 G2 | 249 | 746 | TCC to TGC | Ser to Cys |
| 1210, bladder | carc., T3 G2 | 249 | 746 | TCC to TGC | Ser to Cys |
| 4.13, cervix | carc., stage II | 249 | 746 | TCC to TGC | Ser to Cys |
| 4.139, cervix | carc., stage II | 249 | 746 | TCC to TGC | Ser to Cys |
| 6.96.1, cervix | carc., stage II | 249 | 746 | TCC to TGC | Ser to Cys |

| | | | | | |
|---|---|---|---|---|---|
| Histopathol., histopathological classification of the tumours (carc., carcinoma: TNM and HUGO classifications are used respectively for bladder and cervix cancers) ; codon and mutated nucleotide (Nt position) are numbered according to FGFR3-IIIb cDNA open reading frame. | | | | | |

Mutations were found in the following exons
- exon 7, encoding the junction between immunoglobulin-like domains II and III of FGFR3 (one C-to-T transition at codon 248 in patient 745.1 and a C-to-G substitution at codon 249 in patient 1447) ;
- exon 10, encoding the transmembrane domain (a G-to-T-transversion at codon 372 in patient 813)
- exon 15, encoding the tyrosine kinase domain II (a A-to-G transition at codon 652 in patient 1084).

Analysis of matched constitutional DNA from the patients for which such material was available (n=8) demonstrated the somatic nature of these *FGFR3* mutations (Figure 1).

Strikingly, each of the FGFR3 missense mutations identified herein, i.e. R248C. S249C. G372C and K652E. are implicated in thanatophoric dysplasia (TD). Given the presence of two additional amino-acids in the IIIb isoform expressed in epithelial cancers as compared to the IIIc isoform expressed in bone, the G372C and K652E mutations are indeed equivalent to the G370C and K650E mutations responsible for TD.

The S249C mutation was the most commonly observed, affecting 5 out of 9 (55 %) bladder cancers and all of the cervical cancers (3 out of 3, 100 %) in which *FGFR3* gene alterations have been identified so far.

The R248C, S249C and G372/370C mutations create cysteine residues in the extracellular or transmembrane domains of the receptor and the K652/650E mutations results in amino-acid substitution in the kinase domain of the receptor.

### Example 2 : Inhibitors

A way to test the different FGFR3 inhibitors comprises transfecting cell lines so that they express the mutated forms of FGFR3, or wild type FGFR3 or just the neomycin or hygromycin resistant gene under the control of a strong promoter, such as CMV, RSV, SV40 promoters. The tumorigenic properties of these cell lines can then be compared *in vitro* or *in vivo* in nude mice. The different inhibitors will be tested *in vitro* or *in vivo* using these different cell lines. Phosphorylation, proliferation or indirect effects of FGFR3 such as calcium influx will be measured. Transgenic mice expressing in various epithelia the mutated FGFR3 can thus be derived thereof. Those mice developping tumours are useful tools for testing the efficiency of candidate inhibiting drugs. Such transgenic animals fall also into the scope of the present invention.

### Example 3 : FGFR3 mutations in Ta, T1 tumours in bladder cancer.

Bladder cancer is a disease with a spectrum of forms and is highly unpredictable. At the time of initial diagnosis, approximately 80% of patients present with a superficial tumour. Superficial bladder cancers include carcinoma *in situ* (Tis), Ta and T1 lesions (TNM classification). Ta/T1 lesions are mostly papillary urothelial carcinomas: Ta lesions do not invade the basement membrane, whereas T1 lesions invade the lamina propria, but do not invade the detrusor muscle of the bladder wall. Carcinoma *in situ* are flat, cytologically high-grade carcinomas, confined to the urothelium. Primary isolated carcinoma *in situ* is a very rare entity and is more commonly associated with Ta/T1 lesions. Despite transurethral resection alone or combined with adjuvant intravesical therapies, more than one half of patients with Ta/T1 tumours suffer recurrences. In most cases, recurrences are also superficial, but about 5% of Ta and 30-50% of T1 tumours progress in an unpredictable manner to muscle invasion with a high risk of development of metastases and death from bladder cancer.

The management of superficial bladder cancer is based on clinicopathological parameters. Three groups of tumours can be defined. of low, intermediate and high risk, according to their potential for recurrence and progression. This classification is used to recommend adjuvant intravesical therapies and bladder monitoring, but it is not a sufficiently sensitive discriminant for use in determining the appropriate treatment and mode of surveillance for a given patient. Although Bacille Calmette-Guérin (BCG) therapy appeared to be the most effective regimen for the high-risk group, long-term results indicate that progression occurs in 40% by 10 years and in more than 50% by 15 years. For some researchers, these findings justified the use of up-front radical cystectomy in high-risk superficial urothelial carcinomas, despite the risk of overtreating a significant number of patients. Follow-up of Ta and T1 superficial bladder cancers constitutes most of the workload of urologists involved in the management of bladder cancer. The current strategy is based on frequent cystoscopic evaluations using a schedule that is largely empirical, without considering the individual characteristics of the tumour.

The limitations of the current management of bladder cancer demonstrate the need for prognostic markers, making possible the use of selective aggressive treatments for patients at high risk of progression while sparing low-risk patients from unnecessary procedures. A number of chromosomal loci and specific genes have been implicated in bladder tumorigenesis. Losses of all or part of chromosome 9 in many TaG1 tumours suggests that the inactivation of a gene or genes on chromosome 9 may be an early event in urothelial transformation. The prognostic significance of losses on chromosome 9 is unclear. Alterations of the *P53* and *RB* genes controlling the G1 cell cycle checkpoint have been clearly delineated and are associated with the aggressiveness of superficial and invasive bladder cancers. Despite these recent insights into the molecular mechanisms of bladder carcinoma progression, these markers have not yet had any impact on clinical practice.

The following assays have been performed to assess the reliability, as markers, of the FGFR3 mutations.

### Material and method

### Patients and tissue samples

Seventy four specimens of superficial Ta, T1 bladder carcinomas were obtained from 74 patients by transurethral resection performed at the Henri Mondor hospital, Créteil, France, from January 1988 to December 1998. Tumours were staged according to the TNM classification (1) and graded according to criteria recommended by the World Health Organisation (2). This series consisted of 25 pTa and 49 pT1 tumours, with 28 grade G1, 33 grade G2 and 13 grade G3 tumours. The 64 men and 10 women had a mean age of 64 years (range: 29 to 94 years). None of the patients had any detectable distant metastases at the time of transurethral resection. Patients were treated by transurethral resection (TUR) alone (n=25), TUR followed by mitomycin C instillation (n=10) or TUR and BCG (n=39) according to the French Committee for Urologic Oncology (CCAFU) recommendations. There was no change in the policy for treating superficial bladder cancer during the study period. Progression was defined as the occurrence of a pT2 or higher stage or appearance of lymph node invasion or metastasis or death from cancer. Disease-specific survival curves were plotted using death from urothelial cancer as the endpoint. Follow-up was based on systematic cystoscopy and cytology, and imaging studies only when indicated. All outpatient visits and hospital admissions were recorded in a database from which the study data were calculated.

Tumour DNA was extracted from formalin-fixed and paraffin-embedded tissue or samples freshly frozen in liquid nitrogen (4). Normal DNA samples from peripheral blood were available for 27 patients.

### FGFR3 mutation analysis

Mutations in the *FGFR3* gene were detected by SSCP analysis. Exons 7, 10, 15 and 20 of the *FGFR3* gene were analysed because these exons harbour all the mutations previously identified in bladder carcinomas and thanatophoric dysplasia. All mutations detected by SSCP analysis were confirmed by direct bidirectional sequencing of tumour genomic DNA. Matched normal DNA, if available, was sequenced on both strands to demonstrate the somatic nature of these mutations.

### Statistical methods

Associations between *FGFR3* mutation status and other data (sex, age, stage and grade) were tested using χ² and Student's t tests. Progression-free and disease-specific survival curves were plotted using Kaplan-Meier estimates. Survival distributions were compared using the log-rank test. Cox's proportional hazards regression model was used to test the effect of mutations, while simultaneously accounting for baseline patient and tumour characteristics. The influence of the covariates on the *FGFR3* mutation effect was assessed in multivariate analysis involving a forward stepwise procedure and a backward stepwise procedure, using the MPRL (maximum partial likelihood ratio) method. The limit to enter a term was 0.15 and the limit to remove a term was 0.10. Statistical analyses were performed using BMDP® and S-Plus® software.

### Results

*FGFR3* missense mutations were observed in 41 of the 74 (55%) Ta, T1 bladder tumours. The *FGFR3* mutations found are described in Table 2 below :

**Table 2**

| Number of tumours (%) | Codon* | nt position* | Mutation | Predicted effect |
|---|---|---|---|---|
| 5(12%) | 248 | 742 | CGC -> TGC | Arg -> Cys |
| 28(68.5%) | 249 | 746 | TCC -> TGC | Ser -> Cys |
| 5(12%) | 372 | 1,114 | GGC -> TGC | Gly -> Cys |
| 2(5%) | 375 | 1,124 | TAT -> TGT | Tyr -> Cys |
| 1 (2.5%) | 652 | 1,954 | AAG -> GAG | Lys -> Glu |

| | | | | |
|---|---|---|---|---|
| * codon and mutated nucleotide (nt position) are numbered according to FGFR3-IIIb cDNA open reading frame. FGFR3-IIIb is the isoform expressed in epithelial cells. | | | | |

S249C was the commonest mutation and was found in 16 of the 21 (76%) mutated Ta tumours and 12 of the 20 (60%) mutated T1 tumours. Matched constitutional DNA, available in 15 of the cases of tumour with mutations, contained wild-type sequences, demonstrating the somatic nature of these mutations.

The correlation between sex, age, stage, grade and *FGFR3* mutation status is given Table 3 :

**Table 3**

| | | *FGFR3* wild type | *FGFR3* mutant | p value (χ² or Student's t test) |
|---|---|---|---|---|
| Sex | | | | |
| | Male | 29 | 35 | |
| | Female | 4 | 6 | 0.9779 |

| Age (years) | | | | |
|---|---|---|---|---|
| | mean | 64.30 | 63.22 | |
| | range | [29.15-86.10] | [34.3-94.4] | 0.7393 |

| Stage | | | | |
|---|---|---|---|---|
| | Ta | 4 | 21 | |
| | T1 | 29 | 20 | 0.001 |

| Grade | | | | |
|---|---|---|---|---|
| | G1 | 7 | 21 | |
| | G2 | 14 | 19 | |
| | G3 | 12 | 1 | 0.0003 |

Statistically significant correlations were observed between *FGFR3* mutations and low stage (p=0.001) and low grade (p=0.0003), but not between these mutations and age or sex (Table 2).

With a median follow-up of 4.3 years (range: 6 months to 11 years), 3 patients progressed and one died in the mutated tumour group (n=41 patients) whereas ten patients progressed and eight died in the non-mutated tumour group (n=33 patients). The median follow-up was 5.6 years (range: 7 months to 11 years) in the non-mutated group and 4.1 years (range: 6 months to 9 years) in the mutated group.

To examine *FGFR3* mutations as a marker of patient outcome, we calculated Kaplan-Meier progression-free survival and disease-specific survival probability curves for the two groups of patients and examined the differences using the log rank test. Progression-free and disease-specific survival indicated that *FGFR3* mutations were associated with a lower risk of progression (p=0.014) and longer survival (p=0.007) (Figure 3). We tested several variables (age, sex, stage, grade) but only stage was significantly associated with progression and survival in univariate analysis. If only T1 patients were analysed, the correlation was still significant for disease-specific survival (p=0.03) and close to significance for progression-free survival (p=0.052).

Multivariate analysis was used to determine whether the correlation between *FGFR3* mutation status and progression-free survival or disease-specific survival was independent of other outcome predictors. For progression-free survival, the following covariates were introduced into the Cox model: mutation, stage, grade and sex. For disease-specific survival, mutation and grade were the only covariates introduced into the model, as no disease-related deaths were observed among female or Ta patients. If *FGFR3* status was entered into the model, neither stage nor grade provided any additional prognostic value for tumour progression. In the analysis of disease-specific survival, *FGFR3* mutation was also the only covariate to be entered into the model, as grade did not provide any additional prognostic information. Relative risks and their 95% confidence intervals (CI) are shown in Table 4.

**Table 4**

| | Progression | | Disease-specific Survival | |
|---|---|---|---|---|
| | Relative Risk | 95% CI | Relative Risk | 95% CI |
| *FGFR3* | | | | |
| Wild-type | 1 | | 1 | |
| Mutant | 0.23 | (0.06; 0.83) | 0.10 | (0.01; 0.80) |
| Other variables do not significantly contribute to the model | | | | |

| | | | | |
|---|---|---|---|---|
| Forward and backward procedures both yielded the same model. As shown by the above results, the FGFR3 activating mutations were frequent in bladder carcinomas. | | | | |

All the carcinomas having a mutated receptor expressed said receptor at levels similar or above those observed with normal tissues. Immunohistochemical methods will then advantageously be used for revealing the receptor.

FGFR3 mutation detection in bladder carcinomas appears to be a good pronostic, giving then to the clinicians valuable means for treating and observing carcinomas, which represent a medical problem due to the high frequency of recurrences.

By using SSCP or PCR coupled to an enzymatic restriction S249C mutation specific (which represent 75% of the mutations) on patients having bladder carcinomas with S249C mutation, the mutation could be detected in urine in 60% of the cases.

### Example 4: Detection of FGFR3 mutations in patients' urines

Genomic DNA is extracted from patients' urines and amplified by PCR, in the presence of ³²P- labelled dCTP, using standard methods. The following primers were used for detecting S249C mutation :
5'-CAG CAC CGC CGT CTG GTT GG-3' and 5'-AGT GGC GGT GGT GGT GAG GGA G-3'.
30 cycles of PCR are performed.
The amplification products are digested by *Cac8I.* An additional site is created by *FGFR3* mutation and a corresponding band is observed on an electrophoretic gel.

Similarly the following primers and enzymes can be used to detect:
R248C mutation:
   Primers : 5'-TGT GCG TCA CTG TAC ACC TTG CAG-3' and 5'-AGT GGC GGT GGT GGT GAG GGA G-3'
   Enzyme : *Bsi* HKA I
K652E mutation :
   Primers : 5'-TGG TGA CCG AGG ACA ACG TGA TG-3' and 5'-AGG GTG TGG GAA GGC GGT GTT G-3'
   Enzyme : *Bsm* A I
G372C mutation:
   Primers : 5'-CCT CAA CGC CCA TGT CTT TTC AGC-3' and 5'-CTT GAG CGG GAA GCG GGA GAT CTT G-3'
   Enzyme: *Pst* I
Y375C mutation:
   Primers : 5'-CCT CAA CGC CCA TGT CTT TTC AGC-3' and 5'-CTT GAG CGG GAA GCG GGA GAT CTT G-3'
   Enzyme: *Bsg* I

### References

1. Sobin LH, Fleming ID. TNM Classification of Malignant Tumors, fifth edition (1997). Union Internationale Contre le Cancer and the American Joint Committee on Cancer. Cancer 1997; 80: 1803-4.
2. Epstein JI, Amin MB, Reuter VR, Mostofi FK. The World Health Organization/International Society of Urological Pathology consensus classification of urothelial (transitional cell) neoplasms of the urinary bladder. Bladder Consensus Conference Committee. Am J Surg Pathol 1998; 22: 1435-48.
3. Rischmann P, Bittard H, Chopin D, et al. Tumeurs Urothéliales. Prog Urol 1998; 8: 25-50.
4. Cappellen D, Gil Diez de Medina S, Chopin D, Thiery JP, Radvanyi F. Frequent loss of heterozygosity on chromosome 10q in muscle-invasive transitional cell carcinomas of the bladder. Oncogene 1997; 14: 3059-66.
5. Cappellen D, De Oliveira C, Ricol D, Gil Diez de Medina S, Bourdin J, Sastre-Garau X, Chopin D, Thiery JP, Radvanyi F. Frequent activating mutations of FGFR3 in human bladder and cervix carcinomas. Nature Genetics, vol 23, sept. 1999.
6. Gossen M, Freundlieb S, Bender G, Muller G, Hillen W, Bujard H. Transcriptional activation by tetracyclines in mammalian cells. Science 1995 ; 268 : 1766-9.

## Claims

1. A method for detecting human bladder carcinomas in a biological sample selected from a bladder tissue and urine, comprising identifying FGFR3 activating mutations.

2. The method of claim 1, comprising screening for single nucleotide mutation(s) in nucleic acids of the group comprising genomic DNA, RNA or cDNA.

3. The method of claim 1, comprising screening for single mutation(s) in proteins.

4. The method of claim 1, comprising screening for mutations creating cysteine residues in the extracellular or transmembrane domains of the receptor.

5. The method of claim 1, comprising screening for mutations resulting in at least one aminoacid substitution in the kinase domain of the receptor.

6. The method of claim 1, comprising screening of activating mutation(s) of FGFR3-IIIb.

7. The method of claim 1, comprising screening for mutation(s) in the group comprising exon 7, encoding the junction between immunoglobulin-like domains II and III of FGFR3, exon 10, encoding the transmembrane domain, exon 15, encoding the tyrosine kinase domain I, and the exon encoding the C-terminal part.

8. The method of claim 1, comprising screening for missense mutations such as implicated in thanatophoric dysplasia, NSC, achondroplasia, SADDAN, or hypochondroplasia.

9. The method of claim 8, wherein the mutations comprise R248C, S249C, G372C, S373C, Y375C, K652E, K652M, J809G, J809C, J809R, J809L, P250R, G377C, G382R, A393E, N542K.

10. The method of claim 9, comprising screening R248C, S249C, G372C, K652E and Y375C mutations.

11. Use of an inhibitory antibody directed against FGFR3 for the manufacture of a medicament for treating human bladder carcinomas.

12. Use according to claim 11 wherein the inhibitory antibody is monoclonal.

13. Use according to claim 11 wherein the inhibitory antibody is humanize.

## Patentansprüche

1. Verfahren zum Nachweisen von humanen Blasenkarzinomen in einer biologischen Probe, die aus einem Blasengewebe und Urin ausgewählt ist, umfassend Identifizieren von FGFR3-aktivierenden Mutationen.

2. Verfahren nach Anspruch 1, umfassend ein Screening nach einzelnen Nucleotidmutation(en) in Nucleinsäuren der Gruppe bestehend aus genomischer DNA, RNA oder cDNA.

3. Verfahren nach Anspruch 1, umfassend ein Screening nach einzelnen Mutation(en) in Proteinen.

4. Verfahren nach Anspruch 1, umfassend ein Screening nach Mutationen, die Cysteinreste in den extrazellulären oder transmembranösen Domänen des Rezeptors erzeugen.

5. Verfahren nach Anspruch 1, umfassend ein Screening nach Mutationen, die zu wenigstens einer Aminosäurensubstitution in der Kinasedomäne des Rezeptors führen.

6. Verfahren nach Anspruch 1, umfassend ein Screening nach aktivierenden Mutation(en) von FGFR3-IIIb.

7. Verfahren nach Anspruch 1, umfassend ein Screening nach Mutation(en) in der Gruppe bestehend aus Exon 7, das die Verbindungsstelle zwischen immunoglobulinähnlichen Domänen II und III von FGFR3 codiert, Exon 10, das die transmembranöse Domäne codiert, Exon 15, das die Tyrosinkinasedomäne I codiert, und dem Exon, das den C-terminalen Teil codiert.

8. Verfahren nach Anspruch 1, umfassend ein Screening nach Missense-Mutationen, wie beispielsweise jenen, die an letaler Dysplasie, NSC, Achondroplasie, SADDAN oder Hypochondroplasie beteiligt sind.

9. Verfahren nach Anspruch 8, wobei die Mutationen R248C, S249C, G372C, S373C, Y375C, K652E, K652M, J809G, J809C, J809R, J809L, P250R, G377C, G382R, A393E, N542K umfassen.

10. Verfahren nach Anspruch 9, umfassend ein Screening nach R248C-, S249C-, G372C-, K652E- und Y375C-Mutationen.

11. Verwendung eines inhibitorischen Antikörpers, der gegen FGFR3 gerichtet ist, zur Herstellung eines Arzneimittels zur Behandlung von humanen Blasenkarzinomen.

12. Verwendung nach Anspruch 11, wobei der inhibitorische Antikörper monoklonal ist.

13. Verwendung nach Anspruch 11, wobei der inhibitorische Antikörper humanisiert ist.

## Revendications

1. Procédé de détection de carcinomes humains de la vessie dans un échantillon biologique choisi parmi un tissu de vessie et de l'urine, comprenant l'identification de mutations activatrices du FGFR3.

2. Procédé selon la revendication 1, comprenant le criblage de mutation(s) nucléotidique(s) simple(s) dans des acides nucléiques du groupe comprenant de l'ADN génomique, de l'ARN ou de l'ADNc.

3. Procédé selon la revendication 1, comprenant le criblage de mutation(s) simple(s) dans des protéines.

4. Procédé selon la revendication 1, comprenant le criblage de mutations créant des résidus de cystéine dans les domaines extracellulaires ou transmembranaires du récepteur.

5. Procédé selon la revendication 1, comprenant le criblage de mutations entraînant au moins une substitution d'acide aminé dans le domaine kinase du récepteur.

6. Procédé selon la revendication 1, comprenant le criblage de mutation(s) activatrice(s) du FGFR3-IIIb.

7. Procédé selon la revendication 1, comprenant le criblage de mutation(s) dans le groupe comprenant l'exon 7, codant pour la jonction entre les domaines de type immunoglobuline II et III du FGFR3, l'exon 10, codant pour le domaine transmembranaire, l'exon 15, codant pour le domaine tyrosine kinase I, et l'exon codant pour la partie C-terminale.

8. Procédé selon la revendication 1, comprenant le criblage de mutations faux sens telles qu'impliquées dans la dysplasie thanatophorique, la CNS, l'achondroplasie, le syndrome SADDAN ou l'hypochondroplasie.

9. Procédé selon la revendication 8, dans lequel les mutations comprennent R248C, S249C, G372C, S373C, Y375C, K652E, K652M, J809G, J809C, J809R, J809L, P250R, G377C, G382R, A393E, N542K.

10. Procédé selon la revendication 9, comprenant le criblage des mutations R248C, S249C, G372C, K652E et Y375C.

11. Utilisation d'un anticorps inhibiteur dirigé contre le FGFR3 pour la fabrication d'un médicament destiné au traitement des carcinomes humains de la vessie.

12. Utilisation selon la revendication 11, où l'anticorps inhibiteur est monoclonal.

13. Utilisation selon la revendication 11, où l'anticorps inhibiteur est humanisé.


### LISTE DE SEQUENCES

<110> INSTITUT CURIE
   C.N.R.S.
<120> DETECTION AND TREATMENT OF PATHOLOGIES LINKED TO FGFR3.
<130> 59743-1143
<140> PCT/EP00/04591
   <141> 2000-05-04
<150> US 60/132,705
   <151> 1999-05-05
<160> 20
<170> PatentIn Ver. 2.1
<210> 1
   <211> 2427
   <212> ADN
   <213> Artificial Sequence
<220>
   <223> Description of Artificial Sequence:Wild Type FGFR3-IIIb:
<400> 1
   atgggcgccc ctgcctgcgc cctcgcgctc tgcgtggccg tggccatcgt ggccggcgcc 60
   tcctcggagt ccttggggac ggagcagcgc gtcgtggggc gagcggcaga agtcccgggc 120
   ccagagcccg gccagcagga gcagttggtc ttcggcagcg gggatgctgt ggagctgagc 180
   tgtcccccgc ccgggggtgg tcccatgggg cccactgtct gggtcaagga tggcacaggg 240
   ctggtgccct cggagcgtgt cctggtgggg ccccagcggc tgcaggtgct gaatgcctcc 300
   cacgaggact ccggggccta cagctgccgg cagcggctca cgcagcgcgt actgtgccac 360
   ttcagtgtgc gggtgacaga cgctccatcc tcgggagatg acgaagacgg ggaggacgag 420
   gctgaggaca caggtgtgga cacaggggcc ccttactgga cacggcccga gcggatggac 480
   aagaagctgc tggccgtgcc ggccgccaac accgtccgct tccgctgccc agccgctggc 540
   aaccccactc cctccatctc ctggctgaag aacggcaggg agttccgcgg cgagcaccgc 600
   attggaggca tcaagctgcg gcatcagcag tggagcctgg tcatggaaag cgtggtgccc 660
   tcggaccgcg gcaactacac ctgcgtcgtg gagaacaagt ttggcagcat ccggcagacg 720
   tacacgctgg acgtgctgga gcgctccccg caccggccca tcctgcaggc ggggctgccg 780
   gccaaccaga cggcggtgct gggcagcgac gtggagttcc actgcaaggt gtacagtgac 840
   gcacagcccc acatccagtg gctcaagcac gtggaggtga acggcagcaa ggtgggcccg 900
   gacggcacac cctacgttac cgtgctcaag tcctggatca gtgagagtgt ggaggccgac 960
   gtgcgcctcc gcctggccaa tgtgtcggag cgggacgggg gcgagtacct ctgtcgagcc 1020
   accaatttca taggcgtggc cgagaaggcc ttttggctga gcgttcacgg gccccgagca 1080
   gccgaggagg agctggtgga ggctgacgag gcgggcagtg tgtatgcagg catcctcagc 1140
   tacggggtgg gcttcttcct gttcatcctg gtggtggcgg ctgtgacgct ctgccgcctg 1200
   cgcagccccc ccaagaaagg cctgggctcc cccaccgtgc acaagatctc ccgcttcccg 1260
   ctcaagcgac aggtgtccct ggagtccaac gcgtccatga gctccaacac accactggtg 1320
   cgcatcgcaa ggctgtcctc aggggagggc cccacgctgg ccaatgtctc cgagctcgag 1380
   ctgcctgccg accccaaatg ggagctgtct cgggcccggc tgaccctggg caagcccctt 1440
   ggggagggct gcttcggcca ggtggtcatg gcggaggcca tcggcattga caaggaccgg 1500
   gccgccaagc ctgtcaccgt agccgtgaag atgctgaaag acgatgccac tgacaaggac 1560
   ctgtcggacc tggtgtctga gatggagatg atgaagatga tcgggaaaca caaaaacatc 1620
   atcaacctgc tgggcgcctg cacgcagggc gggcccctgt acgtgctggt ggagtacgcg 1680
   gccaagggta acctgcggga gtttctgcgg gcgcggcggc ccccgggcct ggactactcc 1740
   ttcgacacct gcaagccgcc cgaggagcag ctcaccttca aggacctggt gtcctgtgcc 1800
   taccaggtgg cccggggcat ggagtacttg gcctcccaga agtgcatcca cagggacctg 1860
   gctgcccgca atgtgctggt gaccgaggac aacgtgatga agatcgcaga cttcgggctg 1920
   gcccgggacg tgcacaacct cgactactac aagaagacaa ccaacggccg gctgcccgtg 1980
   aagtggatgg cgcctgaggc cttgtttgac cgagtctaca ctcaccagag tgacgtctgg 2040
   tcctttgggg tcctgctctg ggagatcttc acgctggggg gctccccgta ccccggcatc 2100
   cctgtggagg agctcttcaa gctgctgaag gagggccacc gcatggacaa gcccgccaac 2160
   tgcacacacg acctgtacat gatcatgcgg gagtgctggc atgccgcgcc ctcccagagg 2220
   cccaccttca agcagctggt ggaggacctg gaccgtgtcc ttaccgtgac gtccaccgac 2280
   gagtacctgg acctgtcggc gcctttcgag cagtactccc cgggtggcca ggacaccccc 2340
   agctccagct cctcagggga cgactccgtg tttgcccacg acctgctgcc cccggcccca 2400
   cccagcagtg ggggctcgcg gacgtga                                   2427
<210> 2
   <211> 2427
   <212> ADN
   <213> Artificial Sequence
<220>
   <223> Description of Artificial sequence:Mutant R248C FGFR3-IIIb:
<400> 2
   atgggcgccc ctgcctgcgc cctcgcgctc tgcgtggccg tggccatcgt ggccggcgcc 60
   tcctcggagt ccttggggac ggagcagcgc gtcgtggggc gagcggcaga agtcccgggc 120
   ccagagcccg gccagcagga gcagttggtc ttcggcagcg gggatgctgt ggagctgagc 180
   tgtcccccgc ccgggggtgg tcccatgggg cccactgtct gggtcaagga tggcacaggg 240
   ctggtgccct cggagcgtgt cctggtgggg ccccagcggc tgcaggtgct gaatgcctcc 300
   cacgaggact ccggggccta cagctgccgg cagcggctca cgcagcgcgt actgtgccac 360
   ttcagtgtgc gggtgacaga cgctccatcc tcgggagatg acgaagacgg ggaggacgag 420
   gctgaggaca caggtgtgga cacaggggcc ccttactgga cacggcccga gcggatggac 480
   aagaagctgc tggccgtgcc ggccgccaac accgtccgct tccgctgccc agccgctggc 540
   aaccccactc cctccatctc ctggctgaag aacggcaggg agttccgcgg cgagcaccgc 600
   attggaggca tcaagctgcg gcatcagcag tggagcctgg tcatggaaag cgtggtgccc 660
   tcggaccgcg gcaactacac ctgcgtcgtg gagaacaagt ttggcagcat ccggcagacg 720
   tacacgctgg acgtgctgga gtgctccccg caccggccca tcctgcaggc ggggctgccg 780
   gccaaccaga cggcggtgct gggcagcgac gtggagttcc actgcaaggt gtacagtgac 840
   gcacagcccc acatccagtg gctcaagcac gtggaggtga acggcagcaa ggtgggcccg 900
   gacggcacac cctacgttac cgtgctcaag tcctggatca gtgagagtgt ggaggccgac 960
   gtgcgcctcc gcctggccaa tgtgtcggag cgggacgggg gcgagtacct ctgtcgagcc 1020
   accaatttca taggcgtggc cgagaaggcc ttttggctga gcgttcacgg gccccgagca 1080
   gccgaggagg agctggtgga ggctgacgag gcgggcagtg tgtatgcagg catcctcagc 1140
   tacggggtgg gcttcttcct gttcatcctg gtggtggcgg ctgtgacgct ctgccgcctg 1200
   cgcagccccc ccaagaaagg cctgggctcc cccaccgtgc acaagatctc ccgcttcccg 1260
   ctcaagcgac aggtgtccct ggagtccaac gcgtccatga gctccaacac accactggtg 1320
   cgcatcgcaa ggctgtcctc aggggagggc cccacgctgg ccaatgtctc cgagctcgag 1380
   ctgcctgccg accccaaatg ggagctgtct cgggcccggc tgaccctggg caagcccctt 1440
   ggggagggct gcttcggcca ggtggtcatg gcggaggcca tcggcattga caaggaccgg 1500
   gccgccaagc ctgtcaccgt agccgtgaag atgctgaaag acgatgccac tgacaaggac 1560
   ctgtcggacc tggtgtctga gatggagatg atgaagatga tcgggaaaca caaaaacatc 1620
   atcaacctgc tgggcgcctg cacgcagggc gggcccctgt acgtgctggt ggagtacgcg 1680
   gccaagggta acctgcggga gtttctgcgg gcgcggcggc ccccgggcct ggactactcc 1740
   ttcgacacct gcaagccgcc cgaggagcag ctcaccttca aggacctggt gtcctgtgcc 1800
   taccaggtgg cccggggcat ggagtacttg gcctcccaga agtgcatcca cagggacctg 1860
   gctgcccgca atgtgctggt gaccgaggac aacgtgatga agatcgcaga cttcgggctg 1920
   gcccgggacg tgcacaacct cgactactac aagaagacaa ccaacggccg gctgcccgtg 1980
   aagtggatgg cgcctgaggc cttgtttgac cgagtctaca ctcaccagag tgacgtctgg 2040
   tcctttgggg tcctgctctg ggagatcttc acgctggggg gctccccgta ccccggcatc 2100
   cctgtggagg agctcttcaa gctgctgaag gagggccacc gcatggacaa gcccgccaac 2160
   tgcacacacg acctgtacat gatcatgcgg gagtgctggc atgccgcgcc ctcccagagg 2220
   cccaccttca agcagctggt ggaggacctg gaccgtgtcc ttaccgtgac gtccaccgac 2280
   gagtacctgg acctgtcggc gcctttcgag cagtactccc cgggtggcca ggacaccccc 2340
   agctccagct cctcagggga cgactccgtg tttgcccacg acctgctgcc cccggcccca 2400
   cccagcagtg ggggctcgcg gacgtga 2427
<210> 3
   <211> 2427
   <212> ADN
   <213> Artificial Sequence
<220>
   <223> Description of Artificial Sequence:Mutant S249C PGFR3-IIIb:
<400> 3
   atgggcgccc ctgcctgcgc cctcgcgctc tgcgtggccg tggccatcgt ggccggcgcc 60
   tcctcggagt ccttggggac ggagcagcgc gtcgtggggc gagcggcaga agtcccgggc 120
   ccagagcccg gccagcagga gcagttggtc ttcggcagcg gggatgctgt ggagctgagc 180
   tgtcccccgc ccgggggtgg tcccatgggg cccactgtct gggtcaagga tggcacaggg 240
   ctggtgccct cggagcgtgt cctggtgggg ccccagcggc tgcaggtgct gaatgcctcc 300
   cacgaggact ccggggccta cagctgccgg cagcggctca cgcagcgcgt actgtgccac 360
   ttcagtgtgc gggtgacaga cgctccatcc tcgggagatg acgaagacgg ggaggacgag 420
   gctgaggaca caggtgtgga cacaggggcc ccttactgga cacggcccga gcggatggac 480
   aagaagctgc tggccgtgcc ggccgccaac accgtccgct tccgctgccc agccgctggc 540
   aaccccactc cctccatctc ctggctgaag aacggcaggg agttccgcgg cgagcaccgc 600
   attggaggca tcaagctgcg gcatcagcag tggagcctgg tcatggaaag cgtggtgccc 660
   tcggaccgcg gcaactacac ctgcgtcgtg gagaacaagt ttggcagcat ccggcagacg 720
   tacacgctgg acgtgctgga gcgctgcccg caccggccca tcctgcaggc ggggctgccg 780
   gccaaccaga cggcggtgct gggcagcgac gtggagttcc actgcaaggt gtacagtgac 840
   gcacagcccc acatccagtg gctcaagcac gtggaggtga acggcagcaa ggtgggcccg 900
   gacggcacac cctacgttac cgtgctcaag tcctggatca gtgagagtgt ggaggccgac 960
   gtgcgcctcc gcctggccaa tgtgtcggag cgggacgggg gcgagtacct ctgtcgagcc 1020
   accaatttca taggcgtggc cgagaaggcc ttttggctga gcgttcacgg gccccgagca 1080
   gccgaggagg agctggtgga ggctgacgag gcgggcagtg tgtatgcagg catcctcagc 1140
   tacggggtgg gcttcttcct gttcatcctg gtggtggcgg ctgtgacgct ctgccgcctg 1200
   cgcagccccc ccaagaaagg cctgggctcc cccaccgtgc acaagatctc ccgcttcccg 1260
   ctcaagcgac aggtgtccct ggagtccaac gcgtccatga gctccaacac accactggtg 1320
   cgcatcgcaa ggctgtcctc aggggagggc cccacgctgg ccaatgtctc cgagctcgag 1380
   ctgcctgccg accccaaatg ggagctgtct cgggcccggc tgaccctggg caagcccctt 1440
   ggggagggct gcttcggcca ggtggtcatg gcggaggcca tcggcattga caaggaccgg 1500
   gccgccaagc ctgtcaccgt agccgtgaag atgctgaaag acgatgccac tgacaaggac 1560
   ctgtcggacc tggtgtctga gatggagatg atgaagatga tcgggaaaca caaaaacatc 1620
   atcaacctgc tgggcgcctg cacgcagggc gggcccctgt acgtgctggt ggagtacgcg 1680
   gccaagggta acctgcggga gtttctgcgg gcgcggcggc ccccgggcct ggactactcc 1740
   ttcgacacct gcaagccgcc cgaggagcag ctcaccttca aggacctggt gtcctgtgcc 1800
   taccaggtgg cccggggcat ggagtacttg gcctcccaga agtgcatcca cagggacctg 1860
   gctgcccgca atgtgctggt gaccgaggac aacgtgatga agatcgcaga cttcgggctg 1920
   gcccgggacg tgcacaacct cgactactac aagaagacaa ccaacggccg gctgcccgtg 1980
   aagtggatgg cgcctgaggc cttgtttgac cgagtctaca ctcaccagag tgacgtctgg 2040
   tcctttgggg tcctgctctg ggagatcttc acgctggggg gctccccgta ccccggcatc 2100
   cctgtggagg agctcttcaa gctgctgaag gagggccacc gcatggacaa gcccgccaac 2160
   tgcacacacg acctgtacat gatcatgcgg gagtgctggc atgccgcgcc ctcccagagg 2220
   cccaccttca agcagctggt ggaggacctg gaccgtgtcc ttaccgtgac gtccaccgac 2280
   gagtacctgg acctgtcggc gcctttcgag cagtactccc cgggtggcca ggacaccccc 2340
   agctccagct cctcagggga cgactccgtg tttgcccacg acctgctgcc cccggcccca 2400
   cccagcagtg ggggctcgcg gacgtga                                     2427
<210> 4
   <211> 2427
   <212> ADN
   <213> Artificial Sequence
<220>
   <223> Description of Artificial Sequence:Mutant G372C FGFR3-IIIb:
<400> 4
   atgggcgccc ctgcctgcgc cctcgcgctc tgcgtggccg tggccatcgt ggccggcgcc 60
   tcctcggagt ccttggggac ggagcagcgc gtcgtggggc gagcggcaga agtcccgggc 120
   ccagagcccg gccagcagga gcagttggtc ttcggcagcg gggatgctgt ggagctgagc 180
   tgtcccccgc ccgggggtgg tcccatgggg cccactgtct gggtcaagga tggcacaggg 240
   ctggtgccct cggagcgtgt cctggtgggg ccccagcggc tgcaggtgct gaatgcctcc 300
   cacgaggact ccggggccta cagctgccgg cagcggctca cgcagcgcgt actgtgccac 360
   ttcagtgtgc gggtgacaga cgctccatcc tcgggagatg acgaagacgg ggaggacgag 420
   gctgaggaca caggtgtgga cacaggggcc ccttactgga cacggcccga gcggatggac 480
   aagaagctgc tggccgtgcc ggccgccaac accgtccgct tccgctgccc agccgctggc 540
   aaccccactc cctccatctc ctggctgaag aacggcaggg agttccgcgg cgagcaccgc 600
   attggaggca tcaagctgcg gcatcagcag tggagcctgg tcatggaaag cgtggtgccc 660
   tcggaccgcg gcaactacac ctgcgtcgtg gagaacaagt ttggcagcat ccggcagacg 720
   tacacgctgg acgtgctgga gcgctccccg caccggccca tcctgcaggc ggggctgccg 780
   gccaaccaga cggcggtgct gggcagcgac gtggagttcc actgcaaggt gtacagtgac 840
   gcacagcccc acatccagtg gctcaagcac gtggaggtga acggcagcaa ggtgggcccg 900
   gacggcacac cctacgttac cgtgctcaag tcctggatca gtgagagtgt ggaggccgac 960
   gtgcgcctcc gcctggccaa tgtgtcggag cgggacgggg gcgagtacct ctgtcgagcc 1020
   accaatttca taggcgtggc cgagaaggcc ttttggctga gcgttcacgg gccccgagca 1080
   gccgaggagg agctggtgga ggctgacgag gcgtgcagtg tgtatgcagg catcctcagc 1140
   tacggggtgg gcttcttcct gttcatcctg gtggtggcgg ctgtgacgct ctgccgcctg 1200
   cgcagccccc ccaagaaagg cctgggctcc cccaccgtgc acaagatctc ccgcttcccg 1260
   ctcaagcgac aggtgtccct ggagtccaac gcgtccatga gctccaacac accactggtg 1320
   cgcatcgcaa ggctgtcctc aggggagggc cccacgctgg ccaatgtctc cgagctcgag 1380
   ctgcctgccg accccaaatg ggagctgtct cgggcccggc tgaccctggg caagcccctt 1440
   ggggagggct gcttcggcca ggtggtcatg gcggaggcca tcggcattga caaggaccgg 1500
   gccgccaagc ctgtcaccgt agccgtgaag atgctgaaag acgatgccac tgacaaggac 1560
   ctgtcggacc tggtgtctga gatggagatg atgaagatga tcgggaaaca caaaaacatc 1620
   atcaacctgc tgggcgcctg cacgcagggc gggcccctgt acgtgctggt ggagtacgcg 1680
   gccaagggta acctgcggga gtttctgcgg gcgcggcggc ccccgggcct ggactactcc 1740
   ttcgacacct gcaagccgcc cgaggagcag ctcaccttca aggacctggt gtcctgtgcc 1800
   taccaggtgg cccggggcat ggagtacttg gcctcccaga agtgcatcca cagggacctg 1860
   gctgcccgca atgtgctggt gaccgaggac aacgtgatga agatcgcaga cttcgggctg 1920
   gcccgggacg tgcacaacct cgactactac aagaagacaa ccaacggccg gctgcccgtg 1980
   aagtggatgg cgcctgaggc cttgtttgac cgagtctaca ctcaccagag tgacgtctgg 2040
   tcctttgggg tcctgctctg ggagatcttc acgctggggg gctccccgta ccccggcatc 2100
   cctgtggagg agctcttcaa gctgctgaag gagggccacc gcatggacaa gcccgccaac 2160
   tgcacacacg acctgtacat gatcatgcgg gagtgctggc atgccgcgcc ctcccagagg 2220
   cccaccttca agcagctggt ggaggacctg gaccgtgtcc ttaccgtgac gtccaccgac 2280
   gagtacctgg acctgtcggc gcctttcgag cagtactccc cgggtggcca ggacaccccc 2340
   agctccagct cctcagggga cgactccgtg tttgcccacg acctgctgcc cccggcccca 2400
   cccagcagtg ggggctcgcg gacgtga 2427
<210> 5
   <211> 2427
   <212> ADN
   <213> Artificial Sequence
<220>
   <223> Description of Artificial Sequence:Mutant K652E FGFR3-IIIb:
<400> 5
   atgggcgccc ctgcctgcgc cctcgcgctc tgcgtggccg tggccatcgt ggccggcgcc 60
   tcctcggagt ccttggggac ggagcagcgc gtcgtggggc gagcggcaga agtcccgggc 120
   ccagagcccg gccagcagga gcagttggtc ttcggcagcg gggatgctgt ggagctgagc 180
   tgtcccccgc ccgggggtgg tcccatgggg cccactgtct gggtcaagga tggcacaggg 240
   ctggtgccct cggagcgtgt cctggtgggg ccccagcggc tgcaggtgct gaatgcctcc 300
   cacgaggact ccggggccta cagctgccgg cagcggctca cgcagcgcgt actgtgccac 360
   ttcagtgtgc gggtgacaga cgctccatcc tcgggagatg acgaagacgg ggaggacgag 420
   gctgaggaca caggtgtgga cacaggggcc ccttactgga cacggcccga gcggatggac 480
   aagaagctgc tggccgtgcc ggccgccaac accgtccgct tccgctgccc agccgctggc 540
   aaccccactc cctccatctc ctggctgaag aacggcaggg agttccgcgg cgagcaccgc 600
   attggaggca tcaagctgcg gcatcagcag tggagcctgg tcatggaaag cgtggtgccc 660
   tcggaccgcg gcaactacac ctgcgtcgtg gagaacaagt ttggcagcat ccggcagacg 720
   tacacgctgg acgtgctgga gcgctccccg caccggccca tcctgcaggc ggggctgccg 780
   gccaaccaga cggcggtgct gggcagcgac gtggagttcc actgcaaggt gtacagtgac 840
   gcacagcccc acatccagtg gctcaagcac gtggaggtga acggcagcaa ggtgggcccg 900
   gacggcacac cctacgttac cgtgctcaag tcctggatca gtgagagtgt ggaggccgac 960
   gtgcgcctcc gcctggccaa tgtgtcggag cgggacgggg gcgagtacct ctgtcgagcc 1020
   accaatttca taggcgtggc cgagaaggcc ttttggctga gcgttcacgg gccccgagca 1080
   gccgaggagg agctggtgga ggctgacgag gcgggcagtg tgtatgcagg catcctcagc 1140
   tacggggtgg gcttcttcct gttcatcctg gtggtggcgg ctgtgacgct ctgccgcctg 1200
   cgcagccccc ccaagaaagg cctgggctcc cccaccgtgc acaagatctc ccgcttcccg 1260
   ctcaagcgac aggtgtccct ggagtccaac gcgtccatga gctccaacac accactggtg 1320
   cgcatcgcaa ggctgtcctc aggggagggc cccacgctgg ccaatgtctc cgagctcgag 1380
   ctgcctgccg accccaaatg ggagctgtct cgggcccggc tgaccctggg caagcccctt 1440
   ggggagggct gcttcggcca ggtggtcatg gcggaggcca tcggcattga caaggaccgg 1500
   gccgccaagc ctgtcaccgt agccgtgaag atgctgaaag acgatgccac tgacaaggac 1560
   ctgtcggacc tggtgtctga gatggagatg atgaagatga tcgggaaaca caaaaacatc 1620
   atcaacctgc tgggcgcctg cacgcagggc gggcccctgt acgtgctggt ggagtacgcg 1680
   gccaagggta acctgcggga gtttctgcgg gcgcggcggc ccccgggcct ggactactcc 1740
   ttcgacacct gcaagccgcc cgaggagcag ctcaccttca aggacctggt gtcctgtgcc 1800
   taccaggtgg cccggggcat ggagtacttg gcctcccaga agtgcatcca cagggacctg 1860
   gctgcccgca atgtgctggt gaccgaggac aacgtgatga agatcgcaga cttcgggctg 1920
   gcccgggacg tgcacaacct cgactactac aaggagacaa ccaacggccg gctgcccgtg 1980
   aagtggatgg cgcctgaggc cttgtttgac cgagtctaca ctcaccagag tgacgtctgg 2040
   tcctttgggg tcctgctctg ggagatcttc acgctggggg gctccccgta ccccggcatc 2100
   cctgtggagg agctcttcaa gctgctgaag gagggccacc gcatggacaa gcccgccaac 2160
   tgcacacacg acctgtacat gatcatgcgg gagtgctggc atgccgcgcc ctcccagagg 2220
   cccaccttca agcagctggt ggaggacctg gaccgtgtcc ttaccgtgac gtccaccgac 2280
   gagtacctgg acctgtcggc gcctttcgag cagtactccc cgggtggcca ggacaccccc 2340
   agctccagct cctcagggga cgactccgtg tttgcccacg acctgctgcc cccggcccca 2400
   cccagcagtg ggggctcgcg gacgtga 2427
<210> 6
   <211> 2427
   <212> ADN
   <213> Artificial Sequence
<220>
   <223> Description of Artificial Sequence:Mutant S373C FGFR3-IIIb:
<400> 6
   atgggcgccc ctgcctgcgc cctcgcgctc tgcgtggccg tggccatcgt ggccggcgcc 60
   tcctcggagt ccttggggac ggagcagcgc gtcgtggggc gagcggcaga agtcccgggc 120
   ccagagcccg gccagcagga gcagttggtc ttcggcagcg gggatgctgt ggagctgagc 180
   tgtcccccgc ccgggggtgg tcccatgggg cccactgtct gggtcaagga tggcacaggg 240
   ctggtgccct cggagcgtgt cctggtgggg ccccagcggc tgcaggtgct gaatgcctcc 300
   cacgaggact ccggggccta cagctgccgg cagcggctca cgcagcgcgt actgtgccac 360
   ttcagtgtgc gggtgacaga cgctccatcc tcgggagatg acgaagacgg ggaggacgag 420
   gctgaggaca caggtgtgga cacaggggcc ccttactgga cacggcccga gcggatggac 480
   aagaagctgc tggccgtgcc ggccgccaac accgtccgct tccgctgccc agccgctggc 540
   aaccccactc cctccatctc ctggctgaag aacggcaggg agttccgcgg cgagcaccgc 600
   attggaggca tcaagctgcg gcatcagcag tggagcctgg tcatggaaag cgtggtgccc 660
   tcggaccgcg gcaactacac ctgcgtcgtg gagaacaagt ttggcagcat ccggcagacg 720
   tacacgctgg acgtgctgga gcgctccccg caccggccca tcctgcaggc ggggctgccg 780
   gccaaccaga cggcggtgct gggcagcgac gtggagttcc actgcaaggt gtacagtgac 840
   gcacagcccc acatccagtg gctcaagcac gtggaggtga acggcagcaa ggtgggcccg 900
   gacggcacac cctacgttac cgtgctcaag tcctggatca gtgagagtgt ggaggccgac 960
   gtgcgcctcc gcctggccaa tgtgtcggag cgggacgggg gcgagtacct ctgtcgagcc 1020
   accaatttca taggcgtggc cgagaaggcc ttttggctga gcgttcacgg gccccgagca 1080
   gccgaggagg agctggtgga ggctgacgag gcgggctgtg tgtatgcagg catcctcagc 1140
   tacggggtgg gcttcttcct gttcatcctg gtggtggcgg ctgtgacgct ctgccgcctg 1200
   cgcagccccc ccaagaaagg cctgggctcc cccaccgtgc acaagatctc ccgcttcccg 1260
   ctcaagcgac aggtgtccct ggagtccaac gcgtccatga gctccaacac accactggtg 1320
   cgcatcgcaa ggctgtcctc aggggagggc cccacgctgg ccaatgtctc cgagctcgag 1380
   ctgcctgccg accccaaatg ggagctgtct cgggcccggc tgaccctggg caagcccctt 1440
   ggggagggct gcttcggcca ggtggtcatg gcggaggcca tcggcattga caaggaccgg 1500
   gccgccaagc ctgtcaccgt agccgtgaag atgctgaaag acgatgccac tgacaaggac 1560
   ctgtcggacc tggtgtctga gatggagatg atgaagatga tcgggaaaca caaaaacatc 1620
   atcaacctgc tgggcgcctg cacgcagggc gggcccctgt acgtgctggt ggagtacgcg 1680
   gccaagggta acctgcggga gtttctgcgg gcgcggcggc ccccgggcct ggactactcc 1740
   ttcgacacct gcaagccgcc cgaggagcag ctcaccttca aggacctggt gtcctgtgcc 1800
   taccaggtgg cccggggcat ggagtacttg gcctcccaga agtgcatcca cagggacctg 1860
   gctgcccgca atgtgctggt gaccgaggac aacgtgatga agatcgcaga cttcgggctg 1920
   gcccgggacg tgcacaacct cgactactac aagaagacaa ccaacggccg gctgcccgtg 1980
   aagtggatgg cgcctgaggc cttgtttgac cgagtctaca ctcaccagag tgacgtctgg 2040
   tcctttgggg tcctgctctg ggagatcttc acgctggggg gctccccgta ccccggcatc 2100
   cctgtggagg agctcttcaa gctgctgaag gagggccacc gcatggacaa gcccgccaac 2160
   tgcacacacg acctgtacat gatcatgcgg gagtgctggc atgccgcgcc ctcccagagg 2220
   cccaccttca agcagctggt ggaggacctg gaccgtgtcc ttaccgtgac gtccaccgac 2280
   gagtacctgg acctgtcggc gcctttcgag cagtactccc cgggtggcca ggacaccccc 2340
   agctccagct cctcagggga cgactccgtg tttgcccacg acctgctgcc cccggcccca 2400
   cccagcagtg gggctcgcgg gacgtga                                   2427
<210> 7
   <211> 2427
   <212> ADN
   <213> Artificial Sequence
<220>
   <223> Description of Artificial Sequence:Mutant Y375C FGFR3-IIIb:
<400> 7
   atgggcgccc ctgcctgcgc cctcgcgctc tgcgtggccg tggccatcgt ggccggcgcc 60
   tcctcggagt ccttggggac ggagcagcgc gtcgtggggc gagcggcaga agtcccgggc 120
   ccagagcccg gccagcagga gcagttggtc ttcggcagcg gggatgctgt ggagctgagc 180
   tgtcccccgc ccgggggtgg tcccatgggg cccactgtct gggtcaagga tggcacaggg 240
   ctggtgccct cggagcgtgt cctggtgggg ccccagcggc tgcaggtgct gaatgcctcc 300
   cacgaggact ccggggccta cagctgccgg cagcggctca cgcagcgcgt actgtgccac 360
   ttcagtgtgc gggtgacaga cgctccatcc tcgggagatg acgaagacgg ggaggacgag 420
   gctgaggaca caggtgtgga cacaggggcc ccttactgga cacggcccga gcggatggac 480
   aagaagctgc tggccgtgcc ggccgccaac accgtccgct tccgctgccc agccgctggc 540
   aaccccactc cctccatctc ctggctgaag aacggcaggg agttccgcgg cgagcaccgc 600
   attggaggca tcaagctgcg gcatcagcag tggagcctgg tcatggaaag cgtggtgccc 660
   tcggaccgcg gcaactacac ctgcgtcgtg gagaacaagt ttggcagcat ccggcagacg 720
   tacacgctgg acgtgctgga gcgctccccg caccggccca tcctgcaggc ggggctgccg 780
   gccaaccaga cggcggtgct gggcagcgac gtggagttcc actgcaaggt gtacagtgac 840
   gcacagcccc acatccagtg gctcaagcac gtggaggtga acggcagcaa ggtgggcccg 900
   gacggcacac cctacgttac cgtgctcaag tcctggatca gtgagagtgt ggaggccgac 960
   gtgcgcctcc gcctggccaa tgtgtcggag cgggacgggg gcgagtacct ctgtcgagcc 1020
   accaatttca taggcgtggc cgagaaggcc ttttggctga gcgttcacgg gccccgagca 1080
   gccgaggagg agctggtgga ggctgacgag gcgggcagtg tgtgtgcagg catcctcagc 1140
   tacggggtgg gcttcttcct gttcatcctg gtggtggcgg ctgtgacgct ctgccgcctg 1200
   cgcagccccc ccaagaaagg cctgggctcc cccaccgtgc acaagatctc ccgcttcccg 1260
   ctcaagcgac aggtgtccct ggagtccaac gcgtccatga gctccaacac accactggtg 1320
   cgcatcgcaa ggctgtcctc aggggagggc cccacgctgg ccaatgtctc cgagctcgag 1380
   ctgcctgccg accccaaatg ggagctgtct cgggcccggc tgaccctggg caagcccctt 1440
   ggggagggct gcttcggcca ggtggtcatg gcggaggcca tcggcattga caaggaccgg 1500
   gccgccaagc ctgtcaccgt agccgtgaag atgctgaaag acgatgccac tgacaaggac 1560
   ctgtcggacc tggtgtctga gatggagatg atgaagatga tcgggaaaca caaaaacatc 1620
   atcaacctgc tgggcgcctg cacgcagggc gggcccctgt acgtgctggt ggagtacgcg 1680
   gccaagggta acctgcggga gtttctgcgg gcgcggcggc ccccgggcct ggactactcc 1740
   ttcgacacct gcaagccgcc cgaggagcag ctcaccttca aggacctggt gtcctgtgcc 1800
   taccaggtgg cccggggcat ggagtacttg gcctcccaga agtgcatcca cagggacctg 1860
   gctgcccgca atgtgctggt gaccgaggac aacgtgatga agatcgcaga cttcgggctg 1920
   gcccgggacg tgcacaacct cgactactac aagaagacaa ccaacggccg gctgcccgtg 1980
   aagtggatgg cgcctgaggc cttgtttgac cgagtctaca ctcaccagag tgacgtctgg 2040
   tcctttgggg tcctgctctg ggagatcttc acgctggggg gctccccgta ccccggcatc 2100
   cctgtggagg agctcttcaa gctgctgaag gagggccacc gcatggacaa gcccgccaac 2160
   tgcacacacg acctgtacat gatcatgcgg gagtgctggc atgccgcgcc ctcccagagg 2220
   cccaccttca agcagctggt ggaggacctg gaccgtgtcc ttaccgtgac gtccaccgac 2280
   gagtacctgg acctgtcggc gcctttcgag cagtactccc cgggtggcca ggacaccccc 2340
   agctccagct cctcagggga cgactccgtg tttgcccacg acctgctgcc cccggcccca 2400
   cccagcagtg ggggctcgcg gacgtga 2427
<210> 8
   <211> 2427
   <212> ADN
   <213> Artificial Sequence
<220>
   <223> Description of Artificial Sequence:Mutant K652M FGFR3-IIIb:
<400> 8
   atgggcgccc ctgcctgcgc cctcgcgctc tgcgtggccg tggccatcgt ggccggcgcc 60
   tcctcggagt ccttggggac ggagcagcgc gtcgtggggc gagcggcaga agtcccgggc 120
   ccagagcccg gccagcagga gcagttggtc ttcggcagcg gggatgctgt ggagctgagc 180
   tgtcccccgc ccgggggtgg tcccatgggg cccactgtct gggtcaagga tggcacaggg 240
   ctggtgccct cggagcgtgt cctggtgggg ccccagcggc tgcaggtgct gaatgcctcc 300
   cacgaggact ccggggccta cagctgccgg cagcggctca cgcagcgcgt actgtgccac 360
   ttcagtgtgc gggtgacaga cgctccatcc tcgggagatg acgaagacgg ggaggacgag 420
   gctgaggaca caggtgtgga cacaggggcc ccttactgga cacggcccga gcggatggac 480
   aagaagctgc tggccgtgcc ggccgccaac accgtccgct tccgctgccc agccgctggc 540
   aaccccactc cctccatctc ctggctgaag aacggcaggg agttccgcgg cgagcaccgc 600
   attggaggca tcaagctgcg gcatcagcag tggagcctgg tcatggaaag cgtggtgccc 660
   tcggaccgcg gcaactacac ctgcgtcgtg gagaacaagt ttggcagcat ccggcagacg 720
   tacacgctgg acgtgctgga gcgctccccg caccggccca tcctgcaggc ggggctgccg 780
   gccaaccaga cggcggtgct gggcagcgac gtggagttcc actgcaaggt gtacagtgac 840
   gcacagcccc acatccagtg gctcaagcac gtggaggtga acggcagcaa ggtgggcccg 900
   gacggcacac cctacgttac cgtgctcaag tcctggatca gtgagagtgt ggaggccgac 960
   gtgcgcctcc gcctggccaa tgtgtcggag cgggacgggg gcgagtacct ctgtcgagcc 1020
   accaatttca taggcgtggc cgagaaggcc ttttggctga gcgttcacgg gccccgagca 1080
   gccgaggagg agctggtgga ggctgacgag gcgggcagtg tgtatgcagg catcctcagc 1140
   tacggggtgg gcttcttcct gttcatcctg gtggtggcgg ctgtgacgct ctgccgcctg 1200
   cgcagccccc ccaagaaagg cctgggctcc cccaccgtgc acaagatctc ccgcttcccg 1260
   ctcaagcgac aggtgtccct ggagtccaac gcgtccatga gctccaacac accactggtg 1320
   cgcatcgcaa ggctgtcctc aggggagggc cccacgctgg ccaatgtctc cgagctcgag 1380
   ctgcctgccg accccaaatg ggagctgtct cgggcccggc tgaccctggg caagcccctt 1440
   ggggagggct gcttcggcca ggtggtcatg gcggaggcca tcggcattga caaggaccgg 1500
   gccgccaagc ctgtcaccgt agccgtgaag atgctgaaag acgatgccac tgacaaggac 1560
   ctgtcggacc tggtgtctga gatggagatg atgaagatga tcgggaaaca caaaaacatc 1620
   atcaacctgc tgggcgcctg cacgcagggc gggcccctgt acgtgctggt ggagtacgcg 1680
   gccaagggta acctgcggga gtttctgcgg gcgcggcggc ccccgggcct ggactactcc 1740
   ttcgacacct gcaagccgcc cgaggagcag ctcaccttca aggacctggt gtcctgtgcc 1800
   taccaggtgg cccggggcat ggagtacttg gcctcccaga agtgcatcca cagggacctg 1860
   gctgcccgca atgtgctggt gaccgaggac aacgtgatga agatcgcaga cttcgggctg 1920
   gcccgggacg tgcacaacct cgactactac aagatgacaa ccaacggccg gctgcccgtg 1980
   aagtggatgg cgcctgaggc cttgtttgac cgagtctaca ctcaccagag tgacgtctgg 2040
   tcctttgggg tcctgctctg ggagatcttc acgctggggg gctccccgta ccccggcatc 2100
   cctgtggagg agctcttcaa gctgctgaag gagggccacc gcatggacaa gcccgccaac 2160
   tgcacacacg acctgtacat gatcatgcgg gagtgctggc atgccgcgcc ctcccagagg 2220
   cccaccttca agcagctggt ggaggacctg gaccgtgtcc ttaccgtgac gtccaccgac 2280
   gagtacctgg acctgtcggc gcctttcgag cagtactccc cgggtggcca ggacaccccc 2340
   agctccagct cctcagggga cgactccgtg tttgcccacg acctgctgcc cccggcccca 2400
   cccagcagtg ggggctcgcg gacgtga                                      2427
<210> 9
   <211> 2427
   <212> ADN
   <213> Artificial Sequence
<220>
   <223> Description of Artificial Sequence:Mutant X809C FGFR3-IIIb:
<400> 9
   atgggcgccc ctgcctgcgc cctcgcgctc tgcgtggccg tggccatcgt ggccggcgcc 60
   tcctcggagt ccttggggac ggagcagcgc gtcgtggggc gagcggcaga agtcccgggc 120
   ccagagcccg gccagcagga gcagttggtc ttcggcagcg gggatgctgt ggagctgagc 180
   tgtcccccgc ccgggggtgg tcccatgggg cccactgtct gggtcaagga tggcacaggg 240
   ctggtgccct cggagcgtgt cctggtgggg ccccagcggc tgcaggtgct gaatgcctcc 300
   cacgaggact ccggggccta cagctgccgg cagcggctca cgcagcgcgt actgtgccac 360
   ttcagtgtgc gggtgacaga cgctccatcc tcgggagatg acgaagacgg ggaggacgag 420
   gctgaggaca caggtgtgga cacaggggcc ccttactgga cacggcccga gcggatggac 480
   aagaagctgc tggccgtgcc ggccgccaac accgtccgct tccgctgccc agccgctggc 540
   aaccccactc cctccatctc ctggctgaag aacggcaggg agttccgcgg cgagcaccgc 600
   attggaggca tcaagctgcg gcatcagcag tggagcctgg tcatggaaag cgtggtgccc 660
   tcggaccgcg gcaactacac ctgcgtcgtg gagaacaagt ttggcagcat ccggcagacg 720
   tacacgctgg acgtgctgga gcgctccccg caccggccca tcctgcaggc ggggctgccg 780
   gccaaccaga cggcggtgct gggcagcgac gtggagttcc actgcaaggt gtacagtgac 840
   gcacagcccc acatccagtg gctcaagcac gtggaggtga acggcagcaa ggtgggcccg 900
   gacggcacac cctacgttac cgtgctcaag tcctggatca gtgagagtgt ggaggccgac 960
   gtgcgcctcc gcctggccaa tgtgtcggag cgggacgggg gcgagtacct ctgtcgagcc 1020
   accaatttca taggcgtggc cgagaaggcc ttttggctga gcgttcacgg gccccgagca 1080
   gccgaggagg agctggtgga ggctgacgag gcgggcagtg tgtatgcagg catcctcagc 1140
   tacggggtgg gcttcttcct gttcatcctg gtggtggcgg ctgtgacgct ctgccgcctg 1200
   cgcagccccc ccaagaaagg cctgggctcc cccaccgtgc acaagatctc ccgcttcccg 1260
   ctcaagcgac aggtgtccct ggagtccaac gcgtccatga gctccaacac accactggtg 1320
   cgcatcgcaa ggctgtcctc aggggagggc cccacgctgg ccaatgtctc cgagctcgag 1380
   ctgcctgccg accccaaatg ggagctgtct cgggcccggc tgaccctggg caagcccctt 1440
   ggggagggct gcttcggcca ggtggtcatg gcggaggcca tcggcattga caaggaccgg 1500
   gccgccaagc ctgtcaccgt agccgtgaag atgctgaaag acgatgccac tgacaaggac 1560
   ctgtcggacc tggtgtctga gatggagatg atgaagatga tcgggaaaca caaaaacatc 1620
   atcaacctgc tgggcgcctg cacgcagggc gggcccctgt acgtgctggt ggagtacgcg 1680
   gccaagggta acctgcggga gtttctgcgg gcgcggcggc ccccgggcct ggactactcc 1740
   ttcgacacct gcaagccgcc cgaggagcag ctcaccttca aggacctggt gtcctgtgcc 1800
   taccaggtgg cccggggcat ggagtacttg gcctcccaga agtgcatcca cagggacctg 1860
   gctgcccgca atgtgctggt gaccgaggac aacgtgatga agatcgcaga cttcgggctg 1920
   gcccgggacg tgcacaacct cgactactac aagaagacaa ccaacggccg gctgcccgtg 1980
   aagtggatgg cgcctgaggc cttgtttgac cgagtctaca ctcaccagag tgacgtctgg 2040
   tcctttgggg tcctgctctg ggagatcttc acgctggggg gctccccgta ccccggcatc 2100
   cctgtggagg agctcttcaa gctgctgaag gagggccacc gcatggacaa gcccgccaac 2160
   tgcacacacg acctgtacat gatcatgcgg gagtgctggc atgccgcgcc ctcccagagg 2220
   cccaccttca agcagctggt ggaggacctg gaccgtgtcc ttaccgtgac gtccaccgac 2280
   gagtacctgg acctgtcggc gcctttcgag cagtactccc cgggtggcca ggacaccccc 2340
   agctccagct cctcagggga cgactccgtg tttgcccacg acctgctgcc cccggcccca 2400
   cccagcagtg ggggctcgcg gacgtgc                                     2427
<210> 10
   <211> 2427
   <212> ADN
   <213> Artificial Sequence
<220>
   <223> Description of Artificial Sequence:Mutant X809G FGFR3-IIIb:
   (Mutant 1)
<400> 10
   atgggcgccc ctgcctgcgc cctcgcgctc tgcgtggccg tggccatcgt ggccggcgcc 60
   tcctcggagt ccttggggac ggagcagcgc gtcgtggggc gagcggcaga agtcccgggc 120
   ccagagcccg gccagcagga gcagttggtc ttcggcagcg gggatgctgt ggagctgagc 180
   tgtcccccgc ccgggggtgg tcccatgggg cccactgtct gggtcaagga tggcacaggg 240
   ctggtgccct cggagcgtgt cctggtgggg ccccagcggc tgcaggtgct gaatgcctcc 300
   cacgaggact ccggggccta cagctgccgg cagcggctca cgcagcgcgt actgtgccac 360
   ttcagtgtgc gggtgacaga cgctccatcc tcgggagatg acgaagacgg ggaggacgag 420
   gctgaggaca caggtgtgga cacaggggcc ccttactgga cacggcccga gcggatggac 480
   aagaagctgc tggccgtgcc ggccgccaac accgtccgct tccgctgccc agccgctggc 540
   aaccccactc cctccatctc ctggctgaag aacggcaggg agttccgcgg cgagcaccgc 600
   attggaggca tcaagctgcg gcatcagcag tggagcctgg tcatggaaag cgtggtgccc 660
   tcggaccgcg gcaactacac ctgcgtcgtg gagaacaagt ttggcagcat ccggcagacg 720
   tacacgctgg acgtgctgga gcgctccccg caccggccca tcctgcaggc ggggctgccg 780
   gccaaccaga cggcggtgct gggcagcgac gtggagttcc actgcaaggt gtacagtgac 840
   gcacagcccc acatccagtg gctcaagcac gtggaggtga acggcagcaa ggtgggcccg 900
   gacggcacac cctacgttac cgtgctcaag tcctggatca gtgagagtgt ggaggccgac 960
   gtgcgcctcc gcctggccaa tgtgtcggag cgggacgggg gcgagtacct ctgtcgagcc 1020
   accaatttca taggcgtggc cgagaaggcc ttttggctga gcgttcacgg gccccgagca 1080
   gccgaggagg agctggtgga ggctgacgag gcgggcagtg tgtatgcagg catcctcagc 1140
   tacggggtgg gcttcttcct gttcatcctg gtggtggcgg ctgtgacgct ctgccgcctg 1200
   cgcagccccc ccaagaaagg cctgggctcc cccaccgtgc acaagatctc ccgcttcccg 1260
   ctcaagcgac aggtgtccct ggagtccaac gcgtccatga gctccaacac accactggtg 1320
   cgcatcgcaa ggctgtcctc aggggagggc cccacgctgg ccaatgtctc cgagctcgag 1380
   ctgcctgccg accccaaatg ggagctgtct cgggcccggc tgaccctggg caagcccctt 1440
   ggggagggct gcttcggcca ggtggtcatg gcggaggcca tcggcattga caaggaccgg 1500
   gccgccaagc ctgtcaccgt agccgtgaag atgctgaaag acgatgccac tgacaaggac 1560
   ctgtcggacc tggtgtctga gatggagatg atgaagatga tcgggaaaca caaaaacatc 1620
   atcaacctgc tgggcgcctg cacgcagggc gggcccctgt acgtgctggt ggagtacgcg 1680
   gccaagggta acctgcggga gtttctgcgg gcgcggcggc ccccgggcct ggactactcc 1740
   ttcgacacct gcaagccgcc cgaggagcag ctcaccttca aggacctggt gtcctgtgcc 1800
   taccaggtgg cccggggcat ggagtacttg gcctcccaga agtgcatcca cagggacctg 1860
   gctgcccgca atgtgctggt gaccgaggac aacgtgatga agatcgcaga cttcgggctg 1920
   gcccgggacg tgcacaacct cgactactac aagaagacaa ccaacggccg gctgcccgtg 1980
   aagtggatgg cgcctgaggc cttgtttgac cgagtctaca ctcaccagag tgacgtctgg 2040
   tcctttgggg tcctgctctg ggagatcttc acgctggggg gctccccgta ccccggcatc 2100
   cctgtggagg agctcttcaa gctgctgaag gagggccacc gcatggacaa gcccgccaac 2160
   tgcacacacg acctgtacat gatcatgcgg gagtgctggc atgccgcgcc ctcccagagg 2220
   cccaccttca agcagctggt ggaggacctg gaccgtgtcc ttaccgtgac gtccaccgac 2280
   gagtacctgg acctgtcggc gcctttcgag cagtactccc cgggtggcca ggacaccccc 2340
   agctccagct cctcagggga cgactccgtg tttgcccacg acctgctgcc cccggcccca 2400
   cccagcagtg ggggctcgcg gacggga                                     2427
<210> 11
   <211> 2427
   <212> ADN
   <213> Artificial Sequence
<220>
   <223> Description of Artificial Sequence:Mutant X809G FGFR3-IIIb:
   (Mutant 2)
<400> 11
<210> 12
   <211> 2427
   <212> ADN
   <213> Artificial Sequence
<220>
   <223> Description of Artificial Sequence:Mutant X809G FGFR3-IIIb:
   (Mutant 3)
<400> 12
   atgggcgccc ctgcctgcgc cctcgcgctc tgcgtggccg tggccatcgt ggccggcgcc 60
   tcctcggagt ccttggggac ggagcagcgc gtcgtggggc gagcggcaga agtcccgggc 120
   ccagagcccg gccagcagga gcagttggtc ttcggcagcg gggatgctgt ggagctgagc 180
   tgtcccccgc ccgggggtgg tcccatgggg cccactgtct gggtcaagga tggcacaggg 240
   ctggtgccct cggagcgtgt cctggtgggg ccccagcggc tgcaggtgct gaatgcctcc 300
   cacgaggact ccggggccta cagctgccgg cagcggctca cgcagcgcgt actgtgccac 360
   ttcagtgtgc gggtgacaga cgctccatcc tcgggagatg acgaagacgg ggaggacgag 420
   gctgaggaca caggtgtgga cacaggggcc ccttactgga cacggcccga gcggatggac 480
   aagaagctgc tggccgtgcc ggccgccaac accgtccgct tccgctgccc agccgctggc 540
   aaccccactc cctccatctc ctggctgaag aacggcaggg agttccgcgg cgagcaccgc 600
   attggaggca tcaagctgcg gcatcagcag tggagcctgg tcatggaaag cgtggtgccc 660
   tcggaccgcg gcaactacac ctgcgtcgtg gagaacaagt ttggcagcat ccggcagacg 720
   tacacgctgg acgtgctgga gcgctccccg caccggccca tcctgcaggc ggggctgccg 780
   gccaaccaga cggcggtgct gggcagcgac gtggagttcc actgcaaggt gtacagtgac 840
   gcacagcccc acatccagtg gctcaagcac gtggaggtga acggcagcaa ggtgggcccg 900
   gacggcacac cctacgttac cgtgctcaag tcctggatca gtgagagtgt ggaggccgac 960
   gtgcgcctcc gcctggccaa tgtgtcggag cgggacgggg gcgagtacct ctgtcgagcc 1020
   accaatttca taggcgtggc cgagaaggcc ttttggctga gcgttcacgg gccccgagca 1080
   gccgaggagg agctggtgga ggctgacgag gcgggcagtg tgtatgcagg catcctcagc 1140
   tacggggtgg gcttcttcct gttcatcctg gtggtggcgg ctgtgacgct ctgccgcctg 1200
   cgcagccccc ccaagaaagg cctgggctcc cccaccgtgc acaagatctc ccgcttcccg 1260
   ctcaagcgac aggtgtccct ggagtccaac gcgtccatga gctccaacac accactggtg 1320
   cgcatcgcaa ggctgtcctc aggggagggc cccacgctgg ccaatgtctc cgagctcgag 1380
   ctgcctgccg accccaaatg ggagctgtct cgggcccggc tgaccctggg caagcccctt 1440
   ggggagggct gcttcggcca ggtggtcatg gcggaggcca tcggcattga caaggaccgg 1500
   gccgccaagc ctgtcaccgt agccgtgaag atgctgaaag acgatgccac tgacaaggac 1560
   ctgtcggacc tggtgtctga gatggagatg atgaagatga tcgggaaaca caaaaacatc 1620
   atcaacctgc tgggcgcctg cacgcagggc gggcccctgt acgtgctggt ggagtacgcg 1680
   gccaagggta acctgcggga gtttctgcgg gcgcggcggc ccccgggcct ggactactcc 1740
   ttcgacacct gcaagccgcc cgaggagcag ctcaccttca aggacctggt gtcctgtgcc 1800
   taccaggtgg cccggggcat ggagtacttg gcctcccaga agtgcatcca cagggacctg 1860
   gctgcccgca atgtgctggt gaccgaggac aacgtgatga agatcgcaga cttcgggctg 1920
   gcccgggacg tgcacaacct cgactactac aagaagacaa ccaacggccg gctgcccgtg 1980
   aagtggatgg cgcctgaggc cttgtttgac cgagtctaca ctcaccagag tgacgtctgg 2040
   tcctttgggg tcctgctctg ggagatcttc acgctggggg gctccccgta ccccggcatc 2100
   cctgtggagg agctcttcaa gctgctgaag gagggccacc gcatggacaa gcccgccaac 2160
   tgcacacacg acctgtacat gatcatgcgg gagtgctggc atgccgcgcc ctcccagagg 2220
   cccaccttca agcagctggt ggaggacctg gaccgtgtcc ttaccgtgac gtccaccgac 2280
   gagtacctgg acctgtcggc gcctttcgag cagtactccc cgggtggcca ggacaccccc 2340
   agctccagct cctcagggga cgactccgtg tttgcccacg acctgctgcc cccggcccca 2400
   cccagcagtg ggggctcgcg gacgcga 2427
<210> 13
   <211> 2427
   <212> ADN
   <213> Artificial Sequence
<220>
   <223> Description of Artificial Sequence:Mutant X809L FGFR3-IIIb:
<400> 13
<210> 14
   <211> 2427
   <212> ADN
   <213> Artificial Sequence
<220>
   <223> Description of Artificial Sequence:Mutant N542K FGFR3-IIIb:
   (Mutant 1)
<400> 14
   atgggcgccc ctgcctgcgc cctcgcgctc tgcgtggccg tggccatcgt ggccggcgcc 60
   tcctcggagt ccttggggac ggagcagcgc gtcgtggggc gagcggcaga agtcccgggc 120
   ccagagcccg gccagcagga gcagttggtc ttcggcagcg gggatgctgt ggagctgagc 180
   tgtcccccgc ccgggggtgg tcccatgggg cccactgtct gggtcaagga tggcacaggg 240
   ctggtgccct cggagcgtgt cctggtgggg ccccagcggc tgcaggtgct gaatgcctcc 300
   cacgaggact ccggggccta cagctgccgg cagcggctca cgcagcgcgt actgtgccac 360
   ttcagtgtgc gggtgacaga cgctccatcc tcgggagatg acgaagacgg ggaggacgag 420
   gctgaggaca caggtgtgga cacaggggcc ccttactgga cacggcccga gcggatggac 480
   aagaagctgc tggccgtgcc ggccgccaac accgtccgct tccgctgccc agccgctggc 540
   aaccccactc cctccatctc ctggctgaag aacggcaggg agttccgcgg cgagcaccgc 600
   attggaggca tcaagctgcg gcatcagcag tggagcctgg tcatggaaag cgtggtgccc 660
   tcggaccgcg gcaactacac ctgcgtcgtg gagaacaagt ttggcagcat ccggcagacg 720
   tacacgctgg acgtgctgga gcgctccccg caccggccca tcctgcaggc ggggctgccg 780
   gccaaccaga cggcggtgct gggcagcgac gtggagttcc actgcaaggt gtacagtgac 840
   gcacagcccc acatccagtg gctcaagcac gtggaggtga acggcagcaa ggtgggcccg 900
   gacggcacac cctacgttac cgtgctcaag tcctggatca gtgagagtgt ggaggccgac 960
   gtgcgcctcc gcctggccaa tgtgtcggag cgggacgggg gcgagtacct ctgtcgagcc 1020
   accaatttca taggcgtggc cgagaaggcc ttttggctga gcgttcacgg gccccgagca 1080
   gccgaggagg agctggtgga ggctgacgag gcgggcagtg tgtatgcagg catcctcagc 1140
   tacggggtgg gcttcttcct gttcatcctg gtggtggcgg ctgtgacgct ctgccgcctg 1200
   cgcagccccc ccaagaaagg cctgggctcc cccaccgtgc acaagatctc ccgcttcccg 1260
   ctcaagcgac aggtgtccct ggagtccaac gcgtccatga gctccaacac accactggtg 1320
   cgcatcgcaa ggctgtcctc aggggagggc cccacgctgg ccaatgtctc cgagctcgag 1380
   ctgcctgccg accccaaatg ggagctgtct cgggcccggc tgaccctggg caagcccctt 1440
   ggggagggct gcttcggcca ggtggtcatg gcggaggcca tcggcattga caaggaccgg 1500
   gccgccaagc ctgtcaccgt agccgtgaag atgctgaaag acgatgccac tgacaaggac 1560
   ctgtcggacc tggtgtctga gatggagatg atgaagatga tcgggaaaca caaaaacatc 1620
   atcaaactgc tgggcgcctg cacgcagggc gggcccctgt acgtgctggt ggagtacgcg 1680
   gccaagggta acctgcggga gtttctgcgg gcgcggcggc ccccgggcct ggactactcc 1740
   ttcgacacct gcaagccgcc cgaggagcag ctcaccttca aggacctggt gtcctgtgcc 1800
   taccaggtgg cccggggcat ggagtacttg gcctcccaga agtgcatcca cagggacctg 1860
   gctgcccgca atgtgctggt gaccgaggac aacgtgatga agatcgcaga cttcgggctg 1920
   gcccgggacg tgcacaacct cgactactac aagaagacaa ccaacggccg gctgcccgtg 1980
   aagtggatgg cgcctgaggc cttgtttgac cgagtctaca ctcaccagag tgacgtctgg 2040
   tcctttgggg tcctgctctg ggagatcttc acgctggggg gctccccgta ccccggcatc 2100
   cctgtggagg agctcttcaa gctgctgaag gagggccacc gcatggacaa gcccgccaac 2160
   tgcacacacg acctgtacat gatcatgcgg gagtgctggc atgccgcgcc ctcccagagg 2220
   cccaccttca agcagctggt ggaggacctg gaccgtgtcc ttaccgtgac gtccaccgac 2280
   gagtacctgg acctgtcggc gcctttcgag cagtactccc cgggtggcca ggacaccccc 2340
   agctccagct cctcagggga cgactccgtg tttgcccacg acctgctgcc cccggcccca 2400
   cccagcagtg ggggctcgcg gacgtga 2427
<210> 15
   <211> 2427
   <212> ADN
   <213> Artificial Sequence
<220>
   <223> Description of Artificial Sequence:Mutant N542K FGFR3-IIIb:
   (Mutant 2)
<400> 15
   atgggcgccc ctgcctgcgc cctcgcgctc tgcgtggccg tggccatcgt ggccggcgcc 60
   tcctcggagt ccttggggac ggagcagcgc gtcgtggggc gagcggcaga agtcccgggc 120
   ccagagcccg gccagcagga gcagttggtc ttcggcagcg gggatgctgt ggagctgagc 180
   tgtcccccgc ccgggggtgg tcccatgggg cccactgtct gggtcaagga tggcacaggg 240
   ctggtgccct cggagcgtgt cctggtgggg ccccagcggc tgcaggtgct gaatgcctcc 300
   cacgaggact ccggggccta cagctgccgg cagcggctca cgcagcgcgt actgtgccac 360
   ttcagtgtgc gggtgacaga cgctccatcc tcgggagatg acgaagacgg ggaggacgag 420
   gctgaggaca caggtgtgga cacaggggcc ccttactgga cacggcccga gcggatggac 480
   aagaagctgc tggccgtgcc ggccgccaac accgtccgct tccgctgccc agccgctggc 540
   aaccccactc cctccatctc ctggctgaag aacggcaggg agttccgcgg cgagcaccgc 600
   attggaggca tcaagctgcg gcatcagcag tggagcctgg tcatggaaag cgtggtgccc 660
   tcggaccgcg gcaactacac ctgcgtcgtg gagaacaagt ttggcagcat ccggcagacg 720
   tacacgctgg acgtgctgga gcgctccccg caccggccca tcctgcaggc ggggctgccg 780
   gccaaccaga cggcggtgct gggcagcgac gtggagttcc actgcaaggt gtacagtgac 840
   gcacagcccc acatccagtg gctcaagcac gtggaggtga acggcagcaa ggtgggcccg 900
   gacggcacac cctacgttac cgtgctcaag tcctggatca gtgagagtgt ggaggccgac 960
   gtgcgcctcc gcctggccaa tgtgtcggag cgggacgggg gcgagtacct ctgtcgagcc 1020
   accaatttca taggcgtggc cgagaaggcc ttttggctga gcgttcacgg gccccgagca 1080
   gccgaggagg agctggtgga ggctgacgag gcgggcagtg tgtatgcagg catcctcagc 1140
   tacggggtgg gcttcttcct gttcatcctg gtggtggcgg ctgtgacgct ctgccgcctg 1200
   cgcagccccc ccaagaaagg cctgggctcc cccaccgtgc acaagatctc ccgcttcccg 1260
   ctcaagcgac aggtgtccct ggagtccaac gcgtccatga gctccaacac accactggtg 1320
   cgcatcgcaa ggctgtcctc aggggagggc cccacgctgg ccaatgtctc cgagctcgag 1380
   ctgcctgccg accccaaatg ggagctgtct cgggcccggc tgaccctggg caagcccctt 1440
   ggggagggct gcttcggcca ggtggtcatg gcggaggcca tcggcattga caaggaccgg 1500
   gccgccaagc ctgtcaccgt agccgtgaag atgctgaaag acgatgccac tgacaaggac 1560
   ctgtcggacc tggtgtctga gatggagatg atgaagatga tcgggaaaca caaaaacatc 1620
   atcaagctgc tgggcgcctg cacgcagggc gggcccctgt acgtgctggt ggagtacgcg 1680
   gccaagggta acctgcggga gtttctgcgg gcgcggcggc ccccgggcct ggactactcc 1740
   ttcgacacct gcaagccgcc cgaggagcag ctcaccttca aggacctggt gtcctgtgcc 1800
   taccaggtgg cccggggcat ggagtacttg gcctcccaga agtgcatcca cagggacctg 1860
   gctgcccgca atgtgctggt gaccgaggac aacgtgatga agatcgcaga cttcgggctg 1920
   gcccgggacg tgcacaacct cgactactac aagaagacaa ccaacggccg gctgcccgtg 1980
   aagtggatgg cgcctgaggc cttgtttgac cgagtctaca ctcaccagag tgacgtctgg 2040
   tcctttgggg tcctgctctg ggagatcttc acgctggggg gctccccgta ccccggcatc 2100
   cctgtggagg agctcttcaa gctgctgaag gagggccacc gcatggacaa gcccgccaac 2160
   tgcacacacg acctgtacat gatcatgcgg gagtgctggc atgccgcgcc ctcccagagg 2220
   cccaccttca agcagctggt ggaggacctg gaccgtgtcc ttaccgtgac gtccaccgac 2280
   gagtacctgg acctgtcggc gcctttcgag cagtactccc cgggtggcca ggacaccccc 2340
   agctccagct cctcagggga cgactccgtg tttgcccacg acctgctgcc cccggcccca 2400
   cccagcagtg ggggctcgcg gacgtga 2427
<210> 16
   <211> 2427
   <212> ADN
   <213> Artificial Sequence
<220>
   <223> Description of Artificial Sequence:Mutant G382R FGFR3-IIIb:
   (Mutant 1)
<400> 16
   atgggcgccc ctgcctgcgc cctcgcgctc tgcgtggccg tggccatcgt ggccggcgcc 60
   tcctcggagt ccttggggac ggagcagcgc gtcgtggggc gagcggcaga agtcccgggc 120
   ccagagcccg gccagcagga gcagttggtc ttcggcagcg gggatgctgt ggagctgagc 180
   tgtcccccgc ccgggggtgg tcccatgggg cccactgtct gggtcaagga tggcacaggg 240
   ctggtgccct cggagcgtgt cctggtgggg ccccagcggc tgcaggtgct gaatgcctcc 300
   cacgaggact ccggggccta cagctgccgg cagcggctca cgcagcgcgt actgtgccac 360
   ttcagtgtgc gggtgacaga cgctccatcc tcgggagatg acgaagacgg ggaggacgag 420
   gctgaggaca caggtgtgga cacaggggcc ccttactgga cacggcccga gcggatggac 480
   aagaagctgc tggccgtgcc ggccgccaac accgtccgct tccgctgccc agccgctggc 540
   aaccccactc cctccatctc ctggctgaag aacggcaggg agttccgcgg cgagcaccgc 600
   attggaggca tcaagctgcg gcatcagcag tggagcctgg tcatggaaag cgtggtgccc 660
   tcggaccgcg gcaactacac ctgcgtcgtg gagaacaagt ttggcagcat ccggcagacg 720
   tacacgctgg acgtgctgga gcgctccccg caccggccca tcctgcaggc ggggctgccg 780
   gccaaccaga cggcggtgct gggcagcgac gtggagttcc actgcaaggt gtacagtgac 840
   gcacagcccc acatccagtg gctcaagcac gtggaggtga acggcagcaa ggtgggcccg 900
   gacggcacac cctacgttac cgtgctcaag tcctggatca gtgagagtgt ggaggccgac 960
   gtgcgcctcc gcctggccaa tgtgtcggag cgggacgggg gcgagtacct ctgtcgagcc 1020
   accaatttca taggcgtggc cgagaaggcc ttttggctga gcgttcacgg gccccgagca 1080
   gccgaggagg agctggtgga ggctgacgag gcgggcagtg tgtatgcagg catcctcagc 1140
   tacagggtgg gcttcttcct gttcatcctg gtggtggcgg ctgtgacgct ctgccgcctg 1200
   cgcagccccc ccaagaaagg cctgggctcc cccaccgtgc acaagatctc ccgcttcccg 1260
   ctcaagcgac aggtgtccct ggagtccaac gcgtccatga gctccaacac accactggtg 1320
   cgcatcgcaa ggctgtcctc aggggagggc cccacgctgg ccaatgtctc cgagctcgag 1380
   ctgcctgccg accccaaatg ggagctgtct cgggcccggc tgaccctggg caagcccctt 1440
   ggggagggct gcttcggcca ggtggtcatg gcggaggcca tcggcattga caaggaccgg 1500
   gccgccaagc ctgtcaccgt agccgtgaag atgctgaaag acgatgccac tgacaaggac 1560
   ctgtcggacc tggtgtctga gatggagatg atgaagatga tcgggaaaca caaaaacatc 1620
   atcaacctgc tgggcgcctg cacgcagggc gggcccctgt acgtgctggt ggagtacgcg 1680
   gccaagggta acctgcggga gtttctgcgg gcgcggcggc ccccgggcct ggactactcc 1740
   ttcgacacct gcaagccgcc cgaggagcag ctcaccttca aggacctggt gtcctgtgcc 1800
   taccaggtgg cccggggcat ggagtacttg gcctcccaga agtgcatcca cagggacctg 1860
   gctgcccgca atgtgctggt gaccgaggac aacgtgatga agatcgcaga cttcgggctg 1920
   gcccgggacg tgcacaacct cgactactac aagaagacaa ccaacggccg gctgcccgtg 1980
   aagtggatgg cgcctgaggc cttgtttgac cgagtctaca ctcaccagag tgacgtctgg 2040
   tcctttgggg tcctgctctg ggagatcttc acgctggggg gctccccgta ccccggcatc 2100
   cctgtggagg agctcttcaa gctgctgaag gagggccacc gcatggacaa gcccgccaac 2160
   tgcacacacg acctgtacat gatcatgcgg gagtgctggc atgccgcgcc ctcccagagg 2220
   cccaccttca agcagctggt ggaggacctg gaccgtgtcc ttaccgtgac gtccaccgac 2280
   gagtacctgg acctgtcggc gcctttcgag cagtactccc cgggtggcca ggacaccccc 2340
   agctccagct cctcagggga cgactccgtg tttgcccacg acctgctgcc cccggcccca 2400
   cccagcagtg ggggctcgcg gacgtga                                    2427
<210> 17
   <211> 2427
   <212> ADN
   <213> Artificial Sequence
<220>
   <223> Description of Artificial Sequence:Mutant G382R FGFR3-IIIb:
   (Mutant 2)
<400> 17
   atgggcgccc ctgcctgcgc cctcgcgctc tgcgtggccg tggccatcgt ggccggcgcc 60
   tcctcggagt ccttggggac ggagcagcgc gtcgtggggc gagcggcaga agtcccgggc 120
   ccagagcccg gccagcagga gcagttggtc ttcggcagcg gggatgctgt ggagctgagc 180
   tgtcccccgc ccgggggtgg tcccatgggg cccactgtct gggtcaagga tggcacaggg 240
   ctggtgccct cggagcgtgt cctggtgggg ccccagcggc tgcaggtgct gaatgcctcc 300
   cacgaggact ccggggccta cagctgccgg cagcggctca cgcagcgcgt actgtgccac 360
   ttcagtgtgc gggtgacaga cgctccatcc tcgggagatg acgaagacgg ggaggacgag 420
   gctgaggaca caggtgtgga cacaggggcc ccttactgga cacggcccga gcggatggac 480
   aagaagctgc tggccgtgcc ggccgccaac accgtccgct tccgctgccc agccgctggc 540
   aaccccactc cctccatctc ctggctgaag aacggcaggg agttccgcgg cgagcaccgc 600
   attggaggca tcaagctgcg gcatcagcag tggagcctgg tcatggaaag cgtggtgccc 660
   tcggaccgcg gcaactacac ctgcgtcgtg gagaacaagt ttggcagcat ccggcagacg 720
   tacacgctgg acgtgctgga gcgctccccg caccggccca tcctgcaggc ggggctgccg 780
   gccaaccaga cggcggtgct gggcagcgac gtggagttcc actgcaaggt gtacagtgac 840
   gcacagcccc acatccagtg gctcaagcac gtggaggtga acggcagcaa ggtgggcccg 900
   gacggcacac cctacgttac cgtgctcaag tcctggatca gtgagagtgt ggaggccgac 960
   gtgcgcctcc gcctggccaa tgtgtcggag cgggacgggg gcgagtacct ctgtcgagcc 1020
   accaatttca taggcgtggc cgagaaggcc ttttggctga gcgttcacgg gccccgagca 1080
   gccgaggagg agctggtgga ggctgacgag gcgggcagtg tgtatgcagg catcctcagc 1140
   taccgggtgg gcttcttcct gttcatcctg gtggtggcgg ctgtgacgct ctgccgcctg 1200
   cgcagccccc ccaagaaagg cctgggctcc cccaccgtgc acaagatctc ccgcttcccg 1260
   ctcaagcgac aggtgtccct ggagtccaac gcgtccatga gctccaacac accactggtg 1320
   cgcatcgcaa ggctgtcctc aggggagggc cccacgctgg ccaatgtctc cgagctcgag 1380
   ctgcctgccg accccaaatg ggagctgtct cgggcccggc tgaccctggg caagcccctt 1440
   ggggagggct gcttcggcca ggtggtcatg gcggaggcca tcggcattga caaggaccgg 1500
   gccgccaagc ctgtcaccgt agccgtgaag atgctgaaag acgatgccac tgacaaggac 1560
   ctgtcggacc tggtgtctga gatggagatg atgaagatga tcgggaaaca caaaaacatc 1620
   atcaacctgc tgggcgcctg cacgcagggc gggcccctgt acgtgctggt ggagtacgcg 1680
   gccaagggta acctgcggga gtttctgcgg gcgcggcggc ccccgggcct ggactactcc 1740
   ttcgacacct gcaagccgcc cgaggagcag ctcaccttca aggacctggt gtcctgtgcc 1800
   taccaggtgg cccggggcat ggagtacttg gcctcccaga agtgcatcca cagggacctg 1860
   gctgcccgca atgtgctggt gaccgaggac aacgtgatga agatcgcaga cttcgggctg 1920
   gcccgggacg tgcacaacct cgactactac aagaagacaa ccaacggccg gctgcccgtg 1980
   aagtggatgg cgcctgaggc cttgtttgac cgagtctaca ctcaccagag tgacgtctgg 2040
   tcctttgggg tcctgctctg ggagatcttc acgctggggg gctccccgta ccccggcatc 2100
   cctgtggagg agctcttcaa gctgctgaag gagggccacc gcatggacaa gcccgccaac 2160
   tgcacacacg acctgtacat gatcatgcgg gagtgctggc atgccgcgcc ctcccagagg 2220
   cccaccttca agcagctggt ggaggacctg gaccgtgtcc ttaccgtgac gtccaccgac 2280
   gagtacctgg acctgtcggc gcctttcgag cagtactccc cgggtggcca ggacaccccc 2340
   agctccagct cctcagggga cgactccgtg tttgcccacg acctgctgcc cccggcccca 2400
   cccagcagtg ggggctcgcg gacgtga 2427
<210> 18
   <211> 2427
   <212> ADN
   <213> Artificial Sequence
<220>
   <223> Description of Artificial Sequence:Mutant G377C FGFR3-IIIb:
<400> 18
<210> 19
   <211> 2427
   <212> ADN
   <213> Artificial Sequence
<220>
   <223> Description of Artificial Sequence:Mutant A393E FGFR3-IIIb:
<400> 19
   atgggcgccc ctgcctgcgc cctcgcgctc tgcgtggccg tggccatcgt ggccggcgcc 60
   tcctcggagt ccttggggac ggagcagcgc gtcgtggggc gagcggcaga agtcccgggc 120
   ccagagcccg gccagcagga gcagttggtc ttcggcagcg gggatgctgt ggagctgagc 180
   tgtcccccgc ccgggggtgg tcccatgggg cccactgtct gggtcaagga tggcacaggg 240
   ctggtgccct cggagcgtgt cctggtgggg ccccagcggc tgcaggtgct gaatgcctcc 300
   cacgaggact ccggggccta cagctgccgg cagcggctca cgcagcgcgt actgtgccac 360
   ttcagtgtgc gggtgacaga cgctccatcc tcgggagatg acgaagacgg ggaggacgag 420
   gctgaggaca caggtgtgga cacaggggcc ccttactgga cacggcccga gcggatggac 480
   aagaagctgc tggccgtgcc ggccgccaac accgtccgct tccgctgccc agccgctggc 540
   aaccccactc cctccatctc ctggctgaag aacggcaggg agttccgcgg cgagcaccgc 600
   attggaggca tcaagctgcg gcatcagcag tggagcctgg tcatggaaag cgtggtgccc 660
   tcggaccgcg gcaactacac ctgcgtcgtg gagaacaagt ttggcagcat ccggcagacg 720
   tacacgctgg acgtgctgga gcgctccccg caccggccca tcctgcaggc ggggctgccg 780
   gccaaccaga cggcggtgct gggcagcgac gtggagttcc actgcaaggt gtacagtgac 840
   gcacagcccc acatccagtg gctcaagcac gtggaggtga acggcagcaa ggtgggcccg 900
   gacggcacac cctacgttac cgtgctcaag tcctggatca gtgagagtgt ggaggccgac 960
   gtgcgcctcc gcctggccaa tgtgtcggag cgggacgggg gcgagtacct ctgtcgagcc 1020
   accaatttca taggcgtggc cgagaaggcc ttttggctga gcgttcacgg gccccgagca 1080
   gccgaggagg agctggtgga ggctgacgag gcgggcagtg tgtatgcagg catcctcagc 1140
   tacggggtgg gcttcttcct gttcatcctg gtggtggagg ctgtgacgct ctgccgcctg 1200
   cgcagccccc ccaagaaagg cctgggctcc cccaccgtgc acaagatctc ccgcttcccg 1260
   ctcaagcgac aggtgtccct ggagtccaac gcgtccatga gctccaacac accactggtg 1320
   cgcatcgcaa ggctgtcctc aggggagggc cccacgctgg ccaatgtctc cgagctcgag 1380
   ctgcctgccg accccaaatg ggagctgtct cgggcccggc tgaccctggg caagcccctt 1440
   ggggagggct gcttcggcca ggtggtcatg gcggaggcca tcggcattga caaggaccgg 1500
   gccgccaagc ctgtcaccgt agccgtgaag atgctgaaag acgatgccac tgacaaggac 1560
   ctgtcggacc tggtgtctga gatggagatg atgaagatga tcgggaaaca caaaaacatc 1620
   atcaacctgc tgggcgcctg cacgcagggc gggcccctgt acgtgctggt ggagtacgcg 1680
   gccaagggta acctgcggga gtttctgcgg gcgcggcggc ccccgggcct ggactactcc 1740
   ttcgacacct gcaagccgcc cgaggagcag ctcaccttca aggacctggt gtcctgtgcc 1800
   taccaggtgg cccggggcat ggagtacttg gcctcccaga agtgcatcca cagggacctg 1860
   gctgcccgca atgtgctggt gaccgaggac aacgtgatga agatcgcaga cttcgggctg 1920
   gcccgggacg tgcacaacct cgactactac aagaagacaa ccaacggccg gctgcccgtg 1980
   aagtggatgg cgcctgaggc cttgtttgac cgagtctaca ctcaccagag tgacgtctgg 2040
   tcctttgggg tcctgctctg ggagatcttc acgctggggg gctccccgta ccccggcatc 2100
   cctgtggagg agctcttcaa gctgctgaag gagggccacc gcatggacaa gcccgccaac 2160
   tgcacacacg acctgtacat gatcatgcgg gagtgctggc atgccgcgcc ctcccagagg 2220
   cccaccttca agcagctggt ggaggacctg gaccgtgtcc ttaccgtgac gtccaccgac 2280
   gagtacctgg acctgtcggc gcctttcgag cagtactccc cgggtggcca ggacaccccc 2340
   agctccagct cctcagggga cgactccgtg tttgcccacg acctgctgcc cccggcccca 2400
   cccagcagtg ggggctcgcg gacgtga 2427
<210> 20
   <211> 2427
   <212> ADN
   <213> Artificial Sequence
<220>
   <223> Description of Artificial Sequence:Mutant P250R FGFR3-IIIb:
<400> 20
   atgggcgccc ctgcctgcgc cctcgcgctc tgcgtggccg tggccatcgt ggccggcgcc 60
   tcctcggagt ccttggggac ggagcagcgc gtcgtggggc gagcggcaga agtcccgggc 120
   ccagagcccg gccagcagga gcagttggtc ttcggcagcg gggatgctgt ggagctgagc 180
   tgtcccccgc ccgggggtgg tcccatgggg cccactgtct gggtcaagga tggcacaggg 240
   ctggtgccct cggagcgtgt cctggtgggg ccccagcggc tgcaggtgct gaatgcctcc 300
   cacgaggact ccggggccta cagctgccgg cagcggctca cgcagcgcgt actgtgccac 360
   ttcagtgtgc gggtgacaga cgctccatcc tcgggagatg acgaagacgg ggaggacgag 420
   gctgaggaca caggtgtgga cacaggggcc ccttactgga cacggcccga gcggatggac 480
   aagaagctgc tggccgtgcc ggccgccaac accgtccgct tccgctgccc agccgctggc 540
   aaccccactc cctccatctc ctggctgaag aacggcaggg agttccgcgg cgagcaccgc 600
   attggaggca tcaagctgcg gcatcagcag tggagcctgg tcatggaaag cgtggtgccc 660
   tcggaccgcg gcaactacac ctgcgtcgtg gagaacaagt ttggcagcat ccggcagacg 720
   tacacgctgg acgtgctgga gcgctcccgg caccggccca tcctgcaggc ggggctgccg 780
   gccaaccaga cggcggtgct gggcagcgac gtggagttcc actgcaaggt gtacagtgac 840
   gcacagcccc acatccagtg gctcaagcac gtggaggtga acggcagcaa ggtgggcccg 900
   gacggcacac cctacgttac cgtgctcaag tcctggatca gtgagagtgt ggaggccgac 960
   gtgcgcctcc gcctggccaa tgtgtcggag cgggacgggg gcgagtacct ctgtcgagcc 1020
   accaatttca taggcgtggc cgagaaggcc ttttggctga gcgttcacgg gccccgagca 1080
   gccgaggagg agctggtgga ggctgacgag gcgggcagtg tgtatgcagg catcctcagc 1140
   tacggggtgg gcttcttcct gttcatcctg gtggtggcgg ctgtgacgct ctgccgcctg 1200
   cgcagccccc ccaagaaagg cctgggctcc cccaccgtgc acaagatctc ccgcttcccg 1260
   ctcaagcgac aggtgtccct ggagtccaac gcgtccatga gctccaacac accactggtg 1320
   cgcatcgcaa ggctgtcctc aggggagggc cccacgctgg ccaatgtctc cgagctcgag 1380
   ctgcctgccg accccaaatg ggagctgtct cgggcccggc tgaccctggg caagcccctt 1440
   ggggagggct gcttcggcca ggtggtcatg gcggaggcca tcggcattga caaggaccgg 1500
   gccgccaagc ctgtcaccgt agccgtgaag atgctgaaag acgatgccac tgacaaggac 1560
   ctgtcggacc tggtgtctga gatggagatg atgaagatga tcgggaaaca caaaaacatc 1620
   atcaacctgc tgggcgcctg cacgcagggc gggcccctgt acgtgctggt ggagtacgcg 1680
   gccaagggta acctgcggga gtttctgcgg gcgcggcggc ccccgggcct ggactactcc 1740
   ttcgacacct gcaagccgcc cgaggagcag ctcaccttca aggacctggt gtcctgtgcc 1800
   taccaggtgg cccggggcat ggagtacttg gcctcccaga agtgcatcca cagggacctg 1860
   gctgcccgca atgtgctggt gaccgaggac aacgtgatga agatcgcaga cttcgggctg 1920
   gcccgggacg tgcacaacct cgactactac aagaagacaa ccaacggccg gctgcccgtg 1980
   aagtggatgg cgcctgaggc cttgtttgac cgagtctaca ctcaccagag tgacgtctgg 2040
   tcctttgggg tcctgctctg ggagatcttc acgctggggg gctccccgta ccccggcatc 2100
   cctgtggagg agctcttcaa gctgctgaag gagggccacc gcatggacaa gcccgccaac 2160
   tgcacacacg acctgtacat gatcatgcgg gagtgctggc atgccgcgcc ctcccagagg 2220
   cccaccttca agcagctggt ggaggacctg gaccgtgtcc ttaccgtgac gtccaccgac 2280
   gagtacctgg acctgtcggc gcctttcgag cagtactccc cgggtggcca ggacaccccc 2340
   agctccagct cctcagggga cgactccgtg tttgcccacg acctgctgcc cccggcccca 2400
   cccagcagtg ggggctcgcg gacgtga 2427